# EUROPÄISCHE PATENTSCHRIFT

(11) **EP 1 976 855 B1**
(45) Veröffentlichungstag und Bekanntmachung des Hinweises auf die Patenterteilung: **15.08.2012**
(21) Anmeldenummer: 07702711.8
(22) Anmeldetag: 12.01.2007
(51) Int. Cl.: C07D 498/04

(54) **1-AZA-3,7-DIOXABICYCLO[3.3.0]OCTAN-VERBINDUNGEN UND IHRE VERWENDUNG ALS PRO-FRAGRANCES**
1-AZA-3,7-DIOXABICYCLO[3.3.0]OCTANE COMPOUNDS AND THEIR USE AS PRO-FRAGRANCES
COMPOSES DE 1-AZA-3,7-DIOXABICYCLO[3.3.0]OCTANE ET LEUR UTILISATION EN TANT QUE PRECURSEURS DE PARFUM

(30) Priorität: 20.01.2006 DE 102006003092
(43) Veröffentlichungstag der Anmeldung: 08.10.2008
(73) Patentinhaber: Henkel AG & Co. KGaA, 40589 Düsseldorf (DE)
(72) Erfinder: HUCHEL, Ursula, 50670 Köln (DE); SAUF, Silvia, 40235 Düsseldorf (DE); GERKE, Thomas, 41468 Neuss (DE)
(86) Internationale Anmeldenummer: PCT/EP2007/000238
(87) Internationale Veröffentlichungsnummer: WO 2007/087977

(56) Entgegenhaltungen:
- WO-A-2004/009564
- NOUGUIER, R., CROZET, M.: "Reactivité du Tris(Hydroxymethyl)aminomethane avec des Nitro-Aldehydes Aromatiques. Synthese de Nouveaux Composés Antimicrobiens" TETRAHEDRON LETTERS, Bd. 26, Nr. 45, 1985, Seiten 5523-5524, XP002430334
- PIERCE, J.S. ET AL.: "Tris-(hydroxymethyl)-aminomethane Derivatives. III Oxamides, Ureas, Oxazolidines and 1-Aza-3,7-dioxabicyclo(3.3.0)octanes" JOURNAL OF THE AMERICAN CHEMICAL SOCIETY, 1951, Seiten 2595-2596, XP002430335
- SENKUS, M.: "Some New Derivatives of Amino Hydroxy Compounds" JOURNAL OF THE AMERICAN CHEMICAL SOCIETY, 1945, Seiten 1515-1519, XP002430336

## Beschreibung

Die Erfindung betrifft 1-Aza-3,7-dioxabicyclo[3.3.0]octan-Verbindungen, Verfahren zu ihrer Herstellung, ihre Verwendung als Pro-Fragrances und diese enthaltende Wasch- und Reinigungsmittel, Weichspüler und Kosmetika, sowie ein Verfahren zur Verlängerung des Duftempfindens von derartigen Mitteln.

Neben dem direkten Zusatz von Duftstoffen zu Wasch- und Reinigungsmitteln, Weichspülern und Kosmetika wurde auch der Zusatz so genannter Pro-Fragrances vorgeschlagen. Pro-Fragrances stellen in Analogie zu den Pro-Drugs ein chemisches Derivat eines Duftstoffs dar, das beispielsweise die Flüchtigkeit des Duftstoffs vermindert und unter Umgebungsbedingungen eine zeitlich verzögerte Freisetzung des Duftstoffs erlaubt. Durch eine Derivatisierung von Duftstoffen wie Duftaldehyden oder Duftketonen kann der Dampfdruck dieser Verbindungen erniedrigt werden. Da die Derivatisierungsreaktion reversibel ist, kann der chemisch gebundene Riechaldehyd oder das Riechketon unter bestimmten Bedingungen, beispielsweise Umgebungsbedingungen, an der Bindungsstelle gespalten werden. Dadurch wird der Riechstoff oder Duftstoff wieder freigesetzt, was zu einem verlängerten Dufteindruck führen kann.

DE-A-1 133 847 betrifft die Verwendung der Kondensationsprodukte von Aldehyden und Ketonen mit Oxiaminen in der Parfümerie. Dazu werden die Aldehyde und Ketone mit Ethanolamin oder Diethanolamin umgesetzt.

In der US 6,861,402 sind Pro-Fragrances beschrieben, die einen Duftaldehyd oder ein Duftketon in Form eines Oxazolidins gebunden enthalten. Dabei wird beispielsweise N-Benzolethanolamin mit einem Duftstoff umgesetzt, so dass sich ein monocyclisches Oxazolidin ergibt.

In der US-A-2003/0207786 sind ebenfalls Pro-Fragrances beschrieben, die eine Oxazolidinstruktur aufweisen.

WO 2004/009564 A2 betrifft cyclische Co-Tenside, die durch Kondensationsreaktion von C₃-C₆-Aldehyden mit mehrwertigen Alkoholen, Aminen, Thiolen oder Carbonsäuren entstehen. Die Co-Tenside eignen sich für den Einsatz in Haushaltswaschmitteln, Haushaltsreinigern, Körperreinigungsmitteln und Körperpflegemitteln.

Aufgabe der vorliegenden Erfindung ist es nun, alternative Duftstoff-Vorformen, sogenannte ProFragrances bereitzustellen.

Aufgabe der vorliegenden Erfindung ist weiterhin die Bereitstellung von Pro-Fragrances, die einen verlängerten Dufteindruck bei Duftaldehyden und Duftketonen erlauben, die an sich einen hohen Dampfdruck aufweisen.

Insbesondere bestand die Aufgabe darin, hydrolysestabile Profragrances bereitzustellen, die sich auch In wassrlge Wasch- und Reinigungsmittel einarbeiten lassen, ohne bereits Im Produkt übermäßigen Hydrolyseerscheinungen zu unterllegen. Auch die Einarbeitbarkeit der Verbindungen in granulare Wasch- und Reinigungsmittelzusammensetzungen, ohne dass es im Herstellprozess zu Zersetzungen kommt, ist eine Anforderung an die bereitzustellenden Verbindungen. Weiterhin sollen die mit den erfindungsgemäßen Verbindungen behandelten Substraten, einen angenehmen und langanhaltenden Duft erhalten.

Überraschenderweise wurde nun gefunden, dass Duftstoffe, die als 1-Aza-3,7-dioxabicyclo[3.3.0]octan-Verbindungen (bicyclische Oxazolidin-Derivate) derivatisiert vorliegen, auf einfache Weise für den Einsatz in Wasch- und Reinigungsmitteln sowie kosmetischen Zubereitungen sehr gut geeignete Profragrances erhalten werden.

Die Aufgabe wird daher erfindungsgemäß gelöst durch 1-Aza-3,7-dioxabicyclo[3.3.0]octan-Verbindungen der allgemeinen Formel (I) 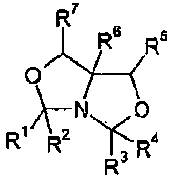 mit der Bedeutung
- R¹, R², R³, R⁴: unabhängig voneinander Reste, die In einer Verbindung der allgemeinen Formel R'-C(=O)-R² bzw. R³-C(=O)-R⁴ einen Duftaldehyd oder ein Duftketon ergeben, wobei R¹ und R² bzw. R³ und R⁴ nicht gleichzeitig Wasserstoff sein können.
- R⁶: H, Alkyl, das durch ein oder zwei Hydroxylgruppen und/oder eine Aminogruppe substituiert sein kann und/oder in dem bis zu 6 nicht benachbarte -CH₂-Gruppen durch - O- ersetzt sein können,
- R⁵, R⁷: unabhängig voneinander H oder C₁₋₆-Alkyl,
oder Gemische dieser Verbindungen, wobei
das Keton ausgewahlt ist aus der Gruppe, bestehend aus Buccoxim; Iso jasmon; Methyl beta naphthyl keton; Moschus indanon; Tonalid/Moschus plus; Alpha-Damascon, Beta-Damascon, Delta-Damascon, Iso-Damascon, Demascenon, Damarose, Methyl-dihydrojasmonat, Menthon, Carvon, Campher, Fenchon, Alpha-Ionen, Beta-Ionon, Dihydro-Beta-Ionon, Gamma-Methyl so genanntes Ionon, Fleuramon, Dihydrojasmon, Cis-Jasmon, Iso-E-Super, Methyl-cedrenyl-keton oder Methyl-cedrylon, Acetophenon, Methyl-acetophenon, Para-Methoxy-acetophenon, Methyl-beta-naphtyl-keton, Benzyl-aceton, Benzophenon, Para-Hydroxy-phenyl-butanon, Celery Keton oder Livescon, 6-Isopropyldecahydro-2-naphton, Dimethyl- octenon, Freskomenth, 4-(1-Ethoxyvinyl)-3,3,5,5,-tetramelhyl-cyclohexanon, Methyl-heptenon, 2-(2-(4-Methyl-3-cyclohexen-1-yl)propyl)-cyclopentanon, 1-(p-Menthen-6(2)-yl)-1-propanon, 4-(4-Hydroxy-3-methoxyphenyl)-2-butanon, 2-Acetyl-3,3-dimethyl-norbornan, 6,7-Dihydro-1,1,2,3,3-pentamethyl, 4(5H)-indanon, 4-Damascol, Dulcinyl or Cassion, Gelson, Hexalon, Isocyclemon E, Methyl-cyclocitron, Methyl-Lavendel-keton, Orivon, Para-tert-butyl-cyclohexanon, Verdon, Delphon. Muscon, Neobutenon, Plicaton, Velouton, 2,4,4,7-Tetramethyl-oct-8-en-3-on, Tetrameran, Hedlon und Gemischen davon. Bevorzugt können die Ketone ausgewählt sein aus Alpha Damascon, Delta Damascon, Iso Damascon, Carvon, Gemma-Methyl-Ionon, Iso-E-Super, 2,4,4,7-Tetramethyl-oct-6-en-3-on, Benzylaceton, Beta Damascon, Damascenon, Methyldihydrojasmonat, Methyl-cedryton, Hedion und Gemischen davon,
und wobei das Aldehyd ausgewählt ist aus Melonal, Triplal, Ligustral, Adoxal; Anisaldehyd; Cymal; Ethylvanillin; Florhydral; Helional; Heliotropin; Hydroxycitronellal; Koavon; Leurinaldehyd; Lyral; Methyl-nonyl-acetaldehyd; p,t-Bucinal; Phenylacetaldehyd; Undecylenaldehyd; Vanillin;2,6,10-Trlmethyl-9-undecenal, 3-Dodecen-I-al, alpha-n-Amylzimlaldehyd, 4-Methoxybenzaldehyd, Benzaldehyd, 3-(4-tert-Butylphenyl)-propanal, 2-Methyl-3-(para-methoxyphenylpropanal), 2-Methyl-4-(2,6,6-trimethyl-2(1)-cyclohexen-1-yl)butanal, 3-Phenyl-2-propenal, cis-/trans-3,7-Dimethyl-2,6-octadien-1-al, 3,7-Dimethyl-6-octen-1-al, [(3,7-Dimethyl-6-octenyl)oxy]acetaldehyd, 4-Isopropylbenzyaldehyd, 1,2,3,4,5,6,7,8-octahydro-8,8-Dimethyl-2-naphthaldehyd, 2,4-Dimethyl-3-cyclohexen-1-carboxyaldehyd, 2-Methyl-3-(isopropylphenyl)propanal,Decyl aldehyd, 2,6-Dimethyl-5-heptenal; 4-(tricyclo[5.2.1.0 (2,6)]-decylidene-8)-butanal; Octahydro-4,7-methano-1H-indenecarboxaldehyd; 3-Ethoxy-4-hydroxybenzaldehyd, para-Ethyl-alpha, alpha-dimethylhydrozimtaldehyd, alpha-Methyl-3,4-(methylenedioxy)-hydrozimtaldehyd, 3,4-Methylenedioxybenzaldehyd, alpha-n-Hexylzimtaldehyd, m-Cymene-7-carboxaldehyd, alpha-Methylphenylacetaldehyd, 7-Hydroxy-3,7-dimethyl octanal, Undecenal, 2,4,6-Trimethyl-3-cyclonexene-1-carboxaldehyd, 4-(3)(4-Methyl-3-pentenyl)-3-cyclohexen-carboxaldehyd, 1-Dodecanal, 2,4-Dimethyl cyclohexene-3-carboxaldehyd, 4-(4-Hydroxy-4-methyl pentyl)-3-cylohexene-1-carboxaldehyd, 7-Methoxy-3,7-dimethyloctan-1-al, 2-Methyl undecanal, 2-Methyl decanal,1-nonanal, 1-Octanal, 2,6,10- Trimethyl-5,9-undecadienal, 2-Methyl-3-(4-tertbutyl)propanal, Dihydrozimtaldehyd, 1-methyl-4-(4-methyl-3-pentenyl)-3-cyclohexene-1-carboxaldehyd, 6 oder 6 Methoxyhexahydro-4,7-methanoindan-1 oder 2-carboxyardehyd, 3,7-Dimethylactan-1-al, 1-Undecanal,10-Undecen-1-al, 4-Hydroxy-3-methoxybenzaldehyd, 1-Methyl-3-(4-methylpentyl)-3-cyclohexenecarboxyaldehyd, 7-Hydroxy-3,7-dimethyl-octanal; trans-4-decenal, 2,6-Nonadienal, para-Tolylacetaldehyd; 4-Methylphenylacetaldehyd, 2-Methyl-4-(2,6,6-trimethyl-1-cyclohexen-1-yl)-2-butenal, ortho-Methoxyzimtaldehyd, 3,5,6-Trimethyl-3-cyclohexenecarboxaldehyd; 3,7-Dimethyl-2-methylene-6-octenal, Phenoxyacetaldehyd; 5,9-Dimethyl-4,8-decadienal, Peony aldehyd (6,10-dimethyl-3-oxa-5,9-undecadien-1-al), Hexahydro-4,7-methanoindan-1-carboxaldehyd, 2-Methyl octanal, alpha-Methyl-4-(1-methylethyl) benzeneacetaldehyd, 6,6-Dimethyl-2-norpinene-2-prapionaldehyd, para Methyl phenoxy acetaldehyd; 2-methyl-3-phenyl-2-propen-1-al, 3,5,5-Trimethylhexanal, Hexahydro-8,8-dimethyl-2-naphthaldehyd, 3-Propyl-bicyclo [2.2.1]-hept-5-ene-2-carbaldehyd, 9-Decenal, 3-Methyl-5-phenyl-1-pentanal, Methylnonyl acetaldehyd, 1-p-Menthene-q-carboxaldehyd, Citral oder Gemische davon, Lillal citral, 1-Decanal, Florhydral, 2,4-Dimethyl-3-cyclohexen-1-carboxaldehyd; bevorzugte Aldehyde können ausgewählt sein aus cis/trans-3,7-Dimethyl-2,6-octadien-I-al; Hellotropin; 2,4,6-Trimethyl-3-cyclohexene-1-carboxaldehyd; 2,6-Nonadlenal; alpha-n-Amylzimtaldehyd, alpha-n-Hexylzimtaldehyd, p-tert Bucinal; Lyral, Cymal, Methylnonylacetaldehyd, trans-2-Nonenal, Lillal, trans-2-Nonenal und Gemische davon.

Die Aufgabe wird zudem gelöst durch Mischungen der Verbindungen der allgemeinen Formel (I) und Verbindungen der allgemeinen Formel (II) 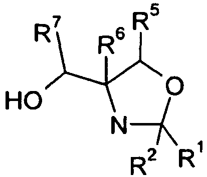 mit den vorstehend angegebenen Bedeutungen für R¹, R², R⁵, R⁶ und R⁷.

Die erfindungsgemäßen Verbindungen der allgemeinen Formel (I) bzw. die diese Verbindungen enthaltenden Mischungen werden erfindungsgemäß als Pro-Fragrances, insbesondere in Waschund Reinigungsmitteln, Weichspülern und Kosmetika eingesetzt.

Die Erfindung betrifft ebenso Wasch- oder Reinigungsmittel, Weichspüler oder Kosmetika, die Verbindungen der allgemeinen Formel (I) oder diese enthaltende Mischungen aufweisen.

Die Erfindung betrifft ferner ein Verfahren zur Verlängerung des Duftempfindens von Wasch- und Reinigungsmitteln, Weichspülern und Kosmetika oder mit diesen behandelten festen Oberflächen, wobei man den Wasch- oder Reinigungsmitteln, Weichspülern oder Kosmetika Verbindungen der allgemeinen Formel (I) oder diese enthaltende Mischungen einverleibt.

Öllösliche substituierte mono- und bicyclische Oxazolidine, die als Additive beispielsweise in Automatikgetriebeflüssigkeiten eingesetzt werden, sind aus der US 4,277,353 bekannt. Beispielhaft werden Umsetzungsprodukte von gegebenenfalls substituierten 2-Amino-1,3-propandiolen mit Paraformaldehyd und Isobutyraldehyd beschrieben. Die Derivatisierung von Duftaldehyden oder Duftketonen ist jedoch nicht erwähnt. Gemäß einer Ausführungsform der vorliegenden Erfindung sind die folgenden Verbindungen ausgenommen: 1-Aza-3,7-dioxa-5-methyl-bicyclo[3.3.0]octan, 1-Aza-3,7-dioxa-5-ethyl-bicyclo[3.3.0]octan, 1-Aza-3,7-dioxabicyclo[3.3.0]octan und 1-Aza-3,7-dioxa-2,8-diisopropyl-5-ethyl-bicyclo[3.3.0]octan. Ferner sind gemäß einer Ausführungsform der vorliegenden Erfindung R¹ und R³ keine C₁₋₃₀-Hydrocarbylreste, sofern R² und R⁴ und R⁵ und R⁷ Wasserstoff sind und R⁶ Wasserstoff, Methyl oder Ethyl bedeutet. Ferner sind gemäß einer Ausführungsform der Erfindung Verbindungen ausgeschlossen, in denen in dem Strukturelement - CR¹R² der Rest R¹ ein C₁₋₃₀-Hydrocarbylrest ist und R² Wasserstoff ist, und in dem Strukturelement -CR³R⁴ die Reste R³ und R⁴ jeweils C₁₋₇-Hydrocarbylreste darstellen.

Es wurde erfindungsgemäß gefunden, dass bicyclische Oxazolidin-Derivate von Duftaldehyden und Duftketonen eine Verminderung des Dampfdrucks der Duftaldehyde und Duftketone und eine Verlängerung des Dufteindrucks erlauben. Zudem kann die Ablagerung der bicyclischen Verbindungen auf festen Oberflächen wie Textilien, Haut oder harten Oberflächen verbessert werden.

Die erfindungsgemäßen Verbindungen der allgemeinen Formel (I) werden erhalten durch Umsetzung von Verbindungen der allgemeinen Formel (III) 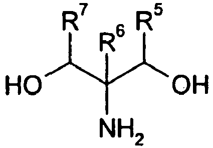 mit Verbindungen der allgemeinen Formeln R¹-C(=O)-R² und R³-C(=O)-R⁴ unter Ringschluss.

Die Verbindungen der allgemeinen Formel (III) leiten sich von 2-Amino-1,3-propandiol ab. Durch Herstellung der bicyclischen Verbindungen ist es möglich, einen hohen Beladungsgrad der 2-Amino-1,3-propandiole zu erreichen, so dass der Einsatz geringerer Mengen der 2-Amino-1,3-propandiole erforderlich ist. Damit kann eine Verlängerung des Dufteindrucks bereits mit geringeren Mengen an 2-Amino-1,3-propandiolen erreicht werden, was unter anderem zu Kostenvorteilen führen kann und auch den Eintrag größerer Chemikalienmengen in Wasch- oder Reinigungsmittel, Weichspüler oder Kosmetika vermeidet.

Es ist erfindungsgemäß auch möglich, Gemische von einfach und zweifach geschlossenen Verbindungen auf Grundlage der 2-Amino-1,3-propandiole einzusetzen. Dabei ist es bevorzugt, einen hohen Beladungsgrad der 2-Amino-1,3-propandiole zu erreichen, so dass bevorzugt zweifach umgesetzte 2-Amino-1,3-propandiole eingesetzt werden.

In den Verbindungen der allgemeinen Formel (I) bedeuten R¹, R², R³ und R⁴ unabhängig voneinander Reste, die in einer Verbindung der allgemeinen Formel R¹-C(=O)-R² bzw. R³-C(=O)-R⁴ einen Duftaldehyd oder ein Duftketon ergeben. Dabei können R¹ und R² bzw. R³ und R⁴ nicht gleichzeitig Wasserstoff sein. Vorzugsweise weisen in dem Strukturelement -CR¹R² die Reste R¹ und R² und in dem Strukturelement -CR³R⁴ die Reste R³ und R⁴ jeweils zusammen mindestens sechs C-Atome, vorzugsweise mindestens fünf C-Atome, besonders bevorzugt mindestens 4 C-Atome auf.

Erfingdungsgemäß liegen maximal in einem der Strukturelemente -CR¹R² bzw. -CR³R⁴ Reste R¹ und R² bzw. R³ und R⁴ vor, die in einer Verbindung der allgemeinen Formel R¹-C(=O)-R² bzw. R³-C(=O)-R⁴ ein Duftketon ergeben.

In einer bevorzugten Ausführungsform bedeuten R², R⁴, R⁵, R⁶, R⁷ Wasserstoff und R¹ und R³ jeweils einen C₄₋₂₄-Kohlenwasserstoffrest bedeuten.

In den Verbindungen der allgemeinen Formel (I) liegen damit Duftaldehyde und/oder Duftketone vor, die mil 2-Amino-1,3-propandiolen der allgemeinen Formel (II) umgesetzt sind. Als Duftaldehyde oder Duftketone können dabei alle üblichen Duftaldehyde und Duftketone eingesetzt werden, die typischerweise zur Herbeiführung eines angenehmen Duftempfindens eingesetzt werden. Geeignete Duftaldehyde und Duftketone sind dem Fachmann bekannt und beispielsweise in US-A-2003/0158079, Absätze [0154] und [0155] beschrieben. Es kann ferner auf US 6,861,402 verwlesen werden.

Es können auch Gemische unterschiedlicher Ketone eingesetzt werden.

Es kann sich auch um einzelne Aldehyde oder Aldehydgemische handeln.

In der allgemeinsten Form kann zur Herstellung der Verbindungen der allgemeinen Formel (I) ein 2-Amino-1,3-propandiol mit Aldehyden, Ketonen oder Mischungen von Ketonen und Aldehyden umgesetzt werden. Gemäß einer Ausführungsform der Erfindung leiten sich die Verbindungen der allgemeinen Formel (I) von einem 2-Amino-1,3-propandiol -Molekül und zwei Aldehydmolekülen oder einem Aldehydmolekül und einem Ketonmolekül ab. Bei der Umsetzung von geringeren als stöchiometrischen Mengen an Aldehyden und/oder Ketonen liegen im Produktgemisch auch monocyclische Verbindungen vor. Der Anteil an bicyclischen Verbindungen zu monocyclischen Verbindungen kann in einfacher Weise durch Auswahl des Molverhältnisses zwischen Aldehyd/Keton und 2-Amino-1,3-propandiol eingestellt werden. Besonders bevorzugt sind hohe Anteile an bicyclischen Strukturen. Vorzugsweise enthalten derartige Mischungen mindestens 50 Gew.-%, vorzugsweise mindestens 65 Gew.-%, insbesondere mindestens 80 Gew.-% an bicyclischen Strukturen.

Die Herstellung der erfindungsgemäßen Verbindungen der allgemeinen Formel (I) erfolgt ausgehend von 2-Amino-1,3-propandiolen der allgemeinen Formel (III). Dabei kann R³ Wasserstoff oder Alkyl bedeuten, das durch ein oder zwei Hydroxylgruppen und/oder eine Aminogruppe substituiert sein kann, wobei auch bis zu 8 nicht benachbarte -CH₂-Gruppen durch -O- ersetzt sein können. Alkylreste sind dabei bevorzugt C₁₋₂₄-Alkylreste, besonders bevorzugt C₁₋₁₆-Alkylreste, insbesondere C₁₋₁₂-Alkylreste, speziell C₁₋₆-Alkylreste, beispielhaft C₁₋₃-Alkylreste. Alkylreste können dabei linear, verzweigt oder cyclisch sein. Vorzugsweise handelt es sich um lineare Alkylreste. Es kann sich um Mono- oder Dihydroxyalkylreste handeln, die anstelle der Hydroxylgruppen oder zusätzlich auch eine Aminogruppe aufweisen können. Die Alkylreste können ferner substituiert oder unsubstituiert sein. Sofern die Alkylreste durch -0- unterbrochen sind, handelt es sich vorzugsweise um Strukturelemente der Formel -CH₂-CH₂-O- oder -CH₂-CH(CH₃)- . O-. Derartige Verbindungen sind in einfacher Weise durch Alkoxylierung der entsprechenden Hydroxyverbindungen zugänglich.

Besonders bevorzugte Reste R⁶ sind Methyl-, Ethyl-, und Hydroxymethylreste.

R⁵ und R⁷ sind Wasserstoff oder ein C₁₋₆-Alkylrest, vorzugsweise C₁₋₃-Alkylrest. Besonders bevorzugt sind R⁵ und R⁷ Wasserstoff oder ein Methyl- oder Ethylrest, insbesondere Wasserstoff.

Die Herstellung der erfindungsgemäßen Verbindungen der allgemeinen Formel (I) erfolgt durch Umsetzung der Verbindungen der allgemeinen Formel (III) mit Verbindungen der allgemeinen Formeln R¹-C(=O)-R² und R³-C(=O)-R⁴ unter Ringschluss. Die Umsetzung wird dabei vorzugsweise in einem geeigneten Lösungsmittel oder in situ durchgeführt. Geeignete Lösungsmittel sind beispielsweise aromatenhaltige Kohlenwasserstoffe wie Toluol. Die Umsetzung wird dabei vorzugsweise bei einer Temperatur im Bereich von 80 bis 150°C, besonders bevorzugt 100 bis 140 °C durchgeführt. Beispielsweise wird die Verbindung der allgemeinen Formel (III) unter Stickstoffatmosphäre zusammen mit dem gewünschten Keton oder Aldehyd im Lösungsmittel vorgelegt. Sodann wird das Reaklionsgemisch erhitzt, wobei der Feststoff langsam in Lösung geht. Häufig wird sodann unter Rückfluss am Wasserabscheider erhitzt. Das erhaltene Umsetzungsprodukt wird nach üblichen Verfahren isoliert und gegebenenfalls gereinigt.

Die erfindungsgemäßen Verbindungen der allgemeinen Formel (I) oder die diese enthaltenden Mischungen werden erfindungsgemäß als Pro-Fragrances eingesetzt. Der Begriff "Pro-Fragrance" beschreibt dabei Derivate von Duftaldehyden und Duftketonen, die unter Umgebungsbedingungen die ursprünglichen Duftaldehyde und Duftketone freisetzen. Umgebungsbedingungen sind dabei die typischen Umgebungsbedingungen im menschlichen Lebensraum bzw. die auf der menschlichen Haut anzutreffenden Bedingungen. Unter Umgebungsbedingungen zerfallen die Verbindungen der allgemeinen Formel (I) in Umkehr des Herstellungsverfahrens langsam unter Freiselzung der ursprünglichen Duftaldehyde und/oder Duflketone. Die chemisch gebundenen Riechaldehyde und Riechketone werden an der Bindungsstelle gespalten, wodurch die Riechstoffe wieder freigesetzt werden.

Demgemäß ist die Verwendung der erfindungsgemäßen Verbindung als Pro-Fragrances, welche Duftstorfe Duftsloffaldehyde oder Duftstoffketone freisetzt, besonders bevorzugt.

Die erfindungsgemäßen Pro-Fragrances können als alleiniger Duftstoff eingesetzt werden, es ist aber auch möglich. Duftstoffgemische einzusetzen, die lediglich zu einem Teil aus den erfindungsgemäßen Pro-Fragrances bestehen. So können Insbesondere Duftstoffgemische eingesetzt werden, die 1 bis 50 Gew.-%, vorzugsweise 5 bis 40 Gew.-% und Insbesondere maximal 30 Gew.% an erfindungsgemäßen Pro-Fragrances enthalten. In anderen Ausführungsformen, bei denen insbesondere die verzögerte Duftwirkung der Träger-gebundenen Form genutzt werden soll, werden bei der erfindungsgemäßen Verwendung vorteilhaft mindestens 30 Gew.-%, vorzugsweise mindestens 40 Gew.-% und insbesondere mindestens 50 Gew.-% des insgesamt in einem Mittel enthaltenen Parfüms über die erfindungsgemäßen Pro-Fragrances in das Mittel eingebracht, während die restlichen 70 Gew.-%, vorzugsweise 60 Gew.-% und insbesondere 60 Gew.-% des insgesamt im Mittel enthaltenen Parfüms auf übliche Weise aufgesprüht oder anders in das Mittel eingebracht werden. Die erfindungsgemäße Verwendung kann also vorteilhaft dadurch gekennzeichnet sein, dass die erfindungsgemäßen Pro-Fragrances zusammen mit anderen Duftstoffen eingesetzt werden.

Durch die Aufteilung des Gesamt-Parfümgehaltes eines Mittels, beispielsweise eines Wasch-oder Reinlgungsmittels, in Parfüm, welches in Form der erfindungsgemäßen Pro-Fragrances vorliegt und Parfüm, das herkömmlich eingearbeitet wurde, lässt sich eine Vielzahl von Produktcharakteristiken realisieren, die erst durch die erfindungsgemäße Verwendung möglich werden. So ist es beispielsweise denkbar und möglich, den Gesamt-Parfümgehalt eines Mittels in zwei Portionen x und y aufzuteilen, wobei der Anteil x aus erfindungsgemäßen Pro-Fragrances und der Anteil y aus herkömmlichen Parfümölen besteht.

Die einzige Grenze bei den erfindungsgemäßen Pro-Fragrances ist, dass die Duftstoffe, die über die erfindungsgemäßen Pro-Fragrances eingebracht werden, aus der Gruppe der Duftstoffaldehyde oder -ketone stammen müssen.

Die Duftstoffe, die auf herkömmliche Weise in die Mittel inkorporiert werden können, sind dagegen keinerlei Beschränkungen unterworfen. So können als Parfümöle bzw. Duftstoffe einzelne Riechstoffverbindungen natürlichen oder synthetischen Ursprungs, z.B. vom Typ der Ester, Ether, Aldehyde, Ketone, Alkohole und Kohlenwasserstoffe verwendet werden. Riechstoffverbindungen vom Typ der Ester sind z.B. Benzylacetat, Phenoxyethylisobutyrat, p-tert.-Butylcyclohexylacetat, Linalylacetat, Dimethylbenzylcarbinylacetat (DMBCA), Phenylethylacetat, Benzylacetat, Ethylmethylphenylglycinat, Allylcyclohexylpropionat, Styrallylpropionat, Benzylsalicylat, Cyclohexylsalicylat, Floramat, Melusat und Jasmacyclat. Zu den Ethern zählen beispielsweise Benzylethylether und Ambroxan, zu den Aldehyden z.B. die linearen Alkanale mit 8 - 18 C-Atomen, Citral, Citronellal, Citronellyloxyacetaldehyd, Cyclamenaldehyd, Lilial und Bourgeonal, zu den Ketonen z.B. die Jonone, α-Isomethylionon und Methylcedrylketon, zu den Alkoholen Anethol, Citronellol, Eugenol, Geraniol, Linalool, Phenylethylalkohol und Terpineol, zu den Kohlenwasserstoffen gehören hauptsächlich Terpene wie Limonen und Pinen. Bevorzugt werden jedoch Mischungen verschiedener Riechstoffe verwendet, die gemeinsam eine ansprechende Duftnote erzeugen.

Solche Parfümöle können auch natürliche Riechstoffgemische enthalten, wie sie aus pflanzlichen Quellen zugänglich sind, z.B. Pine-, Citrus-, Jasmin-, Patchouly-, Rosen- oder Ylang-Ylang-Öl. Ebenfalls geeignet sind Muskateller-Salbeiöl, Kamillenöl, Nelkenöl, Melissenöl, Minzöl, Zimtblätteröl, Lindenblütenöl, Wacholderbeeröl, Vetiveröl, Olibanumöl, Galbanumöl und Labdanumöl sowie Orangenblütenöl, Neroliöl, Orangenschalenöl und Sandelholzöl.

Weitere herkömmliche Riechstoffe, die im Rahmen der vorliegenden Erfindung einsetzbar sind, sind beispielsweise die ätherischen Öle wie Angelikawurzelöl, Anisöl, Arnikablütenöl, Basilikumöl, Bayöl, Champacablütenöl, Edeltannenöl, Edeltannenzapfenöl, Elemiöl, Eukalyptusöl, Fenchelöl, Fichtennadelöl, Galbanumöl, Geraniumöl, Gingergrasöl, Guajakholzöl, Gurjunbalsamöl, Helichrysumöl, Ho-Öl, Ingweröl, Irisöl, Kajeputöl, Kalmusöl, Kamillenöl, Kampferöl, Kanagaöl, Kardamomenöl, Kassiaöl, Kiefernnadelöl, Kopaïvabalsamöl, Korianderöl, Krauseminzeöl, Kümmelöl, Kuminöl, Lavendelöl, Lemongrasöl, Limetteöl, Mandarinenöl, Melissenöl, Moschuskörneröl, Myrrhenöl, Nelkenöl, Neroliöl, Niaouliöl, Olibanumöl, Origanumöl, Palmarosaöl, Patchuliöl, Perubalsamöl, Petitgrainöl, Pfefferöl, Pfefferminzöl, Pimentöl, Pine-Öl, Rosenöl, Rosmarinöl, Sandelholzöl, Sellerieöl, Spiköl, Sternanisöl, Terpentinöl, Thujaöl, Thymianöl, Verbenaöl, Vetiveröl, Wacholderbeeröl, Wermutöl, Wintergrünöl, Ylang-Ylang-Öl, Ysop-Öl, Zimtöl, Zimtblätteröl, Zitronellöl, Zitronenöl sowie Zypressenöl sowie Ambrettolid, Ambroxan, α-Amylzimtaldehyd, Anethol, Anisaldehyd, Anisalkohol, Anisol, Anthranilsäuremethylester, Acetophenon, Benzylaceton, Benzaldehyd, Benzoesäureethylester, Benzophenon, Benzylalkohol, Benzylacetat, Benzylbenzoat, Benzylformiat, Benzylvalerianat, Borneol, Bornylacetat, Boisambrene forte, α-Bromstyrol, n-Decylaldehyd, n-Dodecylaldehyd, Eugenol, Eugenolmethylether, Eukalyptol, Farnesol, Fenchon, Fenchylacetat, Geranylacetat, Geranylformiat, Heliotropin, Heptincarbonsäuremethylester, Heptaldehyd, Hydrochinon-Dimethylether, Hydroxyzimtaldehyd, Hydroxyzimtalkohol, Indol, Iron, Isoeugenol, Isoeugenolmethylether, Isosafrol, Jasmon, Kampfer, Karvakrol, Karvon, p-Kresolmethylether, Cumarin, p-Methoxyacetophenon, Methyl-n-amylketon, Methylanthranilsäuremethylester, p-Methylacetophenon, Methylchavikol, p-Methylchinolin, Methyl-β-naphthylketon, Methyl-n-nonylacetaldehyd, Methyl-n-nonylketon, Muskon, β-Naphtholethylether, β-Naphthol-methylether, Nerol, n-Nonylaldehyd, Nonylalkohol, n-Octylaldehyd, p-Oxy-Acetophenon, Pentadekanolid, β-Phenylethylalkohol, Phenylacetaldehyd-Dimethyacetal, Phenylessigsäure, Pulegon, Safrol, Salicylsäureisoamylester, Salicylsäuremethylester, Salicylsäurehexylester, Salicylsäurecyclohexylester, Santalol, Sandelice, Skatol, Terpineol, Thymen, Thymol, Troenan, γ-Undelacton, Vanilin, Veratrumaldehyd, Zimtaldehyd, Zimatalkohol, Zimtsäure, Zimtsäureethylester, Zimtsäurebenzylester, Diphenyloxid, Limonen, Linalool, Linalylacetat und -propionat, Melusat, Menthol, Menthon, Methyl-n-heptenon, Pinen, Phenylacetaldehyd, Terpinylacetat, Citral, Citronellal und Mischungen daraus.

Die erfindungsgemäßen Pro-Fragrances werden bevorzugt in Wasch- und Reinigungsmitteln, Weichspülern und Kosmetika eingesetzt. Es kann sich dabei um feste, gelförmige oder flüssige Formulierungen handeln, wobei feste Formulierungen in Pulver-, Granulat-, Tabletten- oder Tab-Form vorliegen können. Bei flüssigen Formulierungen kann es sich um Lösungen, Emulsionen oder Dispersionen handeln.

Waschmittel können dabei der manuellen oder maschinellen Wäsche von insbesondere Textilien dienen. Es kann sich um Wasch- oder Reinigungsmittel für den industriellen Bereich oder für den Haushaltsbereich handeln. Reinigungsmittel können beispielsweise zur Reinigung harter Oberflächen eingesetzt werden. Es kann sich dabei beispielsweise um Geschirrreinigungsmittel handeln, die zur manuellen oder maschinellen Reinigung von Geschirr eingesetzt werden. Es kann sich auch um übliche Industrie- oder Haushaltsreiniger handeln, mit denen harte Oberflächen wie Möbeloberflächen, Fliesen, Kacheln, Wand- und Bodenbeläge gereinigt werden. Bei Weichspülern handelt es sich insbesondere um Weichspüler, die zur Behandlung von Textilien bei oder nach der Wäsche eingesetzt werden. Bei Kosmetika kann es sich um Pasten, Salben, Cremes, Emulsionen, Lotionen sowie auch Lösungen, insbesondere alkoholische Lösungen handeln, die beispielsweise aus der Feinparfümerie bekannt sind. Die einzelnen Mittel können in jeder beliebigen geeigneten Form aufgebracht werden. Es kann sich beispielsweise um durch Sprühen aufzutragende Mittel handeln. Die erfindungsgemäßen Pro-Fragrances können darüber hinaus zur Überdeckung von Schlechtgerüchen eingesetzt werden, die beispielsweise in Kombination mit anderen Absorptionsmitteln eine gute Haftung an festen Oberflächen aufweisen.

Die Erfindung betrifft auch Wasch- oder Reinigungsmittel, Weichspüler oder Kosmetika, die die erfindungsgemäßen Verbindungen oder Mischungen enthalten. Dabei werden die Verbindungen oder Mischungen in zur Wirkung ausreichender Menge eingesetzt. Typischerweise werden in Endformulierungen, das heißt gebrauchsfertigen Wasch- oder Reinigungsmitteln, Weichspülern oder Kosmetika, Verbindungen der allgemeinen Formel (I) oder diese enthaltende Mischungen in Mengen von weniger als 5 Gew.%, vorzugsweise weniger als 2 Gew.-%, insbesondere weniger als 1 Gew.-% eingesetzt. Typische Inhaltsmengen liegen im Bereich von 0,05 bis 0,5 Gew.%, insbesondere 0,1 bis 0,2 Gew.-%. In der Feinparfümerie kann auch mit hohen Wirkstoffkonzentrationen von bis zu 40 Gew.-% an Duftstoffen gearbeitet werden.

Zusammensetzungen üblicher Wasch- oder Reinigungsmittel, Weichspüler und Kosmetika sind dem Fachmann bekannt.

So können Wasch- und Reinigungsmittel und Weichspüler weitere übliche Inhaltsstoffe von Wasch- und Reinigungsmittel und Weichspüler enthalten, wie beispielsweise Tenside, Buildersubstanzen, Bleichmittel, andere Duftstoffe, Enzyme und andere Aktivstoffe, aber auch Desintegrationshilfsmittel, sogenannte Tablettensprengmittel, um den Zerfall hochverdichteter Tabletten oder Tabs zu erleichtern bzw. die Zerfallszeiten zu verkürzen. Zu den wesentlichen Inhaltsstoffen von Wasch- und Reinigungsmitteln und Weichspüler gehören dabei insbesondere Tenside.

Je nach Einsatzzweck der erfindungsgemäßen Mittel wird man den Tensidgehalt höher oder niedriger wählen. Üblicherweise liegt der Tensidgehalt von Waschmitteln zwischen 10 und 40 Gew.%, vorzugsweise zwischen 12,5 und 30 Gew.% und insbesondere zwischen 15 und 25 Gew.%, während Reinigungsmittel für beispielsweise das maschinelle Geschirrspülen zwischen 0,1 und 10 Gew.%, vorzugsweise zwischen 0,5 und 7,5 Gew.% und insbesondere zwischen 1 und 5 Gew.% Tenside enthalten.

Diese grenzflächenaktiven Substanzen stammen aus der Gruppe der anionischen, nichtionischen, zwitterionischen oder kationischen Tenside, wobei anionische Tenside aus ökonomischen Gründen und aufgrund ihres Leistungsspektrums beim Waschen und Reinigen deutlich bevorzugt sind.

Als anionische Tenside eignen sich prinzipiell alle für die Verwendung am menschlichen Körper geeigneten anionischen oberflächenaktiven Stoffe. Diese sind gekennzeichnet durch eine wasserlöslich machende, anionische Gruppe wie z. B. eine Carboxylat-, Sulfat-, Sulfonat- oder Phosphat-Gruppe und eine lipophile Alkylgruppe mit etwa 8 bis 30 C-Atomen. Zusätzlich können im Molekül Glykol- oder Polyglykolether-Gruppen, Ester-, Ether- und Amidgruppen sowie Hydroxylgruppen enthalten sein. Beispiele für geeignete anionische Tenside sind, jeweils in Form der Natrium-, Kalium- und Ammonium- sowie der Mono-, Di- und Trialkanoiammoniumsalze mit 2 bis 4 C-Atomen in der Alkanolgruppe,
- lineare und verzweigte Fettsäuren mit 8 bis 30 C-Atomen (Seifen),
- Ethercarbonsäuren der Formel R¹³-O-(CH₂-CH₂O)ₓ-CH₂-COOH, in der R¹³ eine lineare Alkylgruppe mit 8 bis 30 C-Atomen und x = 0 oder 1 bis 16 ist,
- Acylsarcoside mit 8 bis 24 C-Atomen in der Acylgruppe,
- Acyltauride mit 8 bis 24 C-Atomen in der Acylgruppe,
- Acylisethionate mit 8 bis 24 C-Atomen in der Acylgruppe,
- Sulfobernsteinsäuremono- und -dialkylester mit 8 bis 24 C-Atomen in der Alkylgruppe und Sulfobernsteinsäuremono-alkylpolyoxyethylester mit 8 bis 24 C-Atomen in der Alkylgruppe und 1 bis 6 Oxyethylgruppen,
- lineare Alkansulfonate mit 8 bis 24 C-Atomen,
- lineare Alpha-Olefinsulfonate mit 8 bis 24 C-Atomen,
- Alpha-Sulfofettsäuremethylester von Fettsäuren mit 8 bis 30 C-Atomen,
- Alkylsulfate und Alkylpolyglykolethersulfate der Formel R¹⁴-O(CH₂-CH₂O)ₓ-OSO₃H, in der R¹⁴ eine bevorzugt lineare Alkylgruppe mit 8 bis 30 C-Atomen und x = 0 oder 1 bis 12 ist,
- Gemische oberflächenaktiver Hydroxysulfonate,
- sulfatierte Hydroxyalkylpolyethylen- und/oder Hydroxyalkylenpropylenglykolether,
- Sulfonate ungesättigter Fettsäuren mit 8 bis 24 C-Atomen und 1 bis 6 Doppelbindungen,
- Ester der Weinsäure und Zitronensäure mit Alkoholen, die Anlagerungsprodukte von etwa 2-15 Molekülen Ethylenoxid und/oder Propylenoxid an Fettalkohole mit 8 bis 22 C-Atomen darstellen,
- Alkyl- und/oder Alkenyletherphosphate der Formel (E1-I), 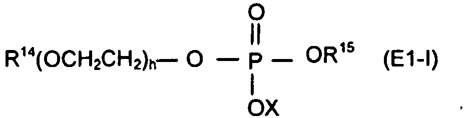 in der R¹⁴ bevorzugt für einen aliphatischen Kohlenwasserstoffrest mit 8 bis 30 Kohlenstoffatomen, R¹⁵ für Wasserstoff, einen Rest (CH₂CH₂O)nR¹⁶ oder X, h für Zahlen von 1 bis 10 und X für Wasserstoff, ein Alkali- oder Erdalkalimetall oder NR¹⁷R¹⁸R¹⁹R²⁰, mit R¹⁷bis R¹⁹ unabhängig voneinander stehend für Wasserstoff oder einen C1 bis C4 -
   Kohlenwasserstoffrest, steht,
- sulfatierte Fettsäurealkylenglykolester der Formel (E1-II)
   R²⁰CO(AlkO)nSO₃M (E1-II)
   in der R²⁰CO- für einen linearen oder verzweigten, aliphatischen, gesättigten und/oder ungesättigten Acylrest mit 6 bis 22 C-Atomen, Alk für CH₂CH₂, CHCH₃CH₂ und/oder CH₂CHCH₃, h für Zahlen von 0,5 bis 5 und M für ein Kation steht,
- Monoglyceridsulfate und Monoglyceridethersulfate der Formel (E1-III) 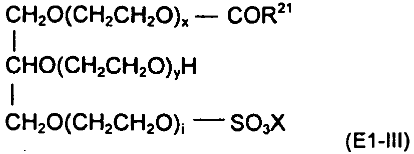 in der R²¹CO für einen linearen oder verzweigten Acylrest mit 6 bis 22 Kohlenstoffatomen, x, y und i in Summe für 0 oder für Zahlen von 1 bis 30, vorzugsweise 2 bis 10, und X für ein Alkali- oder Erdalkalimetall steht. Typische Beispiele für im Sinne der Erfindung geeignete Monoglycerid(ether)sulfate sind die Umsetzungsprodukte von Laurinsäuremonoglycerid, Kokosfettsäuremonoglycerid, Palmitinsäuremonoglycerid, Stearinsäuremonoglycerid, Ölsäuremonoglycerid und Talgfettsäuremonoglycerid sowie deren Ethylenoxidaddukte mit Schwefeltrioxid oder Chlorsulfonsäure in Form ihrer Natriumsalze. Vorzugsweise werden Monoglyceridsulfate der Formel (E1-III) eingesetzt, in der R²¹CO für einen linearen Acylrest mit 8 bis 18 Kohlenstoffatomen steht,
- Amidethercarbonsäuren,
- Kondensationsprodukte aus C₈ - C₃₀ - Fettalkoholen mit Proteinhydrolysaten und/oder Aminosäuren und deren Derivaten, welche dem Fachmann als Eiweissfettsäurekondensate bekannt sind, wie beispielsweise die Lamepon^{®} - Typen, Gluadin^{®} - Typen, Hostapon^{®} KCG oder die Amisoft^{®} - Typen.

Bevorzugte anionische Tenside sind Alkylsulfate, Alkylpolyglykolethersulfate und Ethercarbonsäuren mit 10 bis 18 C-Atomen in der Alkylgruppe und bis zu 12 Glykolethergruppen im Molekül, Sulfobernsteinsäuremono- und -dialkylester mit 8 bis 18 C-Atomen in der Alkylgruppe und Sulfobernsteinsäuremono-alkylpolyoxyethylester mit 8 bis 18 C-Atomen in der Alkylgruppe und 1 bis 6 Oxyethylgruppen, Monoglycerdisulfate, Alkyl- und Alkenyletherphosphate sowie Eiweissfettsäurekondensate.

Ebenfalls können kationische Tenside eingesetzt werden.
Erfindungsgemäß bevorzugt sind kationische Tenside vom Typ der quartären Ammonium-verbindungen, der Esterquats und der Amidoamine. Bevorzugte quartäre Ammoniumverbindungen sind Ammoniumhalogenide, insbesondere Chloride und Bromide, wie Alkyltrimethylammoniumchloride, Dialkyldimethylammoniumchloride und Trialkylmethylammoniumchloride, z.B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid, sowie die unter den INCI-Bezeichnungen Quatemium-27 und Quaternium-83 bekannten Imidazolium-Verbindungen. Die langen Alkylketten der oben genannten Tenside weisen bevorzugt 10 bis 18 Kohlenstoffatome auf.

Bei Esterquats handelt es sich um bekannte Stoffe, die sowohl mindestens eine Esterfunktion als auch mindestens eine quartäre Ammoniumgruppe als Strukturelement enthalten. Bevorzugte Esterquats sind quaternierte Estersalze von Fettsäuren mit Triethanolamin, quaternierte Estersalze von Fettsäuren mit Diethanolalkylaminen und quaternierten Estersalzen von Fettsäuren mit 1,2-Dihydroxypropyldialkylaminen. Solche Produkte werden beispielsweise unter den Warenzeichen Stepantex^{®}, Dehyquart® und Armocare^{®} vertrieben. Die Produkte Armocare^{®} VGH-70, ein N, N-Bis(2-Palmitoyloxyethyl)dimethylammoniumchlorid, sowie Dehyquart® F-75, Dehyquart® C-4046, Dehyquart® L80 und Dehyquart® AU-35 sind Beispiele für solche Esterquats.
Die Alkylamidoamine werden üblicherweise durch Amidierung natürlicher oder synthetischer Fettsäuren und Fettsäureschnitte mit Dialkylaminoaminen hergestellt. Eine erfindungsgemäß besonders geeignete Verbindung aus dieser Substanzgruppe stellt das unter der Bezeichnung Tegoamid® S 18 im Handel erhältliche Stearamidopropyl-dimethylamin dar.
Die kationischen Tenside sind in den erfindungsgemäßen Mitteln bevorzugt in Mengen von 0,05 bis 10 Gew.%, bezogen auf die gesamte Anwendungszubereitung, enthalten. Mengen von 0,1 bis 5 Gew.% sind besonders bevorzugt.

Neben oder statt der kationischen Tenside können die Mittel weitere Tenside oder Emulgatoren enthalten, wobei prinzipiell sowohl anionische als auch ampholytische und nichtionische Tenside und alle Arten bekannter Emulgatoren geeignet sind. Die Gruppe der ampholytischen oder auch amphoteren Tenside umfasst zwitterionische Tenside und Ampholyte. Die Tenside können bereits emulgierende Wirkung haben.

Als zwitterionische Tenside werden solche oberflächenaktiven Verbindungen bezeichnet, die im Molekül mindestens eine quartäre Ammoniumgruppe und mindestens eine -COO⁽⁻⁾ - oder -SO₃ ⁽⁻⁾ - Gruppe tragen. Besonders geeignete zwitterionische Tenside sind die sogenannten Betaine wie die N-Alkyl-N,N-dimethylammonium-glycinate, beispielsweise das Kokosalkyl-dimethylammoniumglycinat, N-Acyl-aminopropyl-N,N-dimethylammoniumglycinate, beispielsweise das Kokosacylaminopropyl-dimethylammoniumglycinat, und 2-Alkyl-3-carboxymethyl-3-hydroxyethyl-imidazoline mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der INCI-Bezeichnung Cocamidopropyl Betaine bekannte Fettsäureamid-Derivat.

Unter Ampholyten werden solche oberflächenaktiven Verbindungen verstanden, die außer einer C₈ - C₂₄ - Alkyl- oder -Acylgruppe im Molekül mindestens eine freie Aminogruppe und mindestens eine -COOH- oder -SO₃H-Gruppe enthalten und zur Ausbildung innerer Salze befähigt sind. Beispiele für geeignete Ampholyte sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 24 C-Atomen in der Alkylgruppe. Besonders bevorzugte Ampholyte sind das N-Kokosalkylaminopropionat, das Kokosacylaminoethylaminopropionat und das C₁₂ - C₁₈ - Acylsarcosin, Nichtionische Tenside enthalten als hydrophile Gruppe z.B. eine Polyolgruppe, eine Polyalkylenglykolethergruppe oder eine Kombination aus Polyol- und Polyglykolethergruppe. Solche Verbindungen sind beispielsweise
- Anlagerungsprodukte von 2 bis 50 Mol Ethylenoxid und/oder 1 bis 5 Mol Propylenoxid an lineare und verzweigte Fettalkohole mit 8 bis 30 C-Atomen, an Fettsäuren mit 8 bis 30 C-Atomen und an Alkylphenole mit 8 bis 15 C-Atomen in der Alkylgruppe,
- mit einem Methyl- oder C₂ - C₆ - Alkylrest endgruppenverschlossene Anlagerungsprodukte von 2 bis 50 Mol Ethylenoxid und/oder 1 bis 5 Mol Propylenoxid an lineare und verzweigte Fettalkohole mit 8 bis 30 C-Atomen, an Fettsäuren mit 8 bis 30 C-Atomen und an Alkylphenole mit 8 bis 15 C-Atomen in der Alkylgruppe, wie beispielsweise die unter den Verkaufsbezeichnungen Dehydol^{®} LS, Dehydol^{®} LT (Cognis) erhältlichen Typen,
- C₁₂-C₃₀-Fettsäuremono- und -diester von Anlagerungsprodukten von 1 bis 30 Mol Ethylenoxid an Glycerin,
- Anlagerungsprodukte von 5 bis 60 Mol Ethylenoxid an Rizinusöl und gehärtetes Rizinusöl,
- Polyolfettsäureester, wie beispielsweise das Handelsprodukt Hydagen^{®} HSP (Cognis) oder Sovermol - Typen (Cognis),
- alkoxilierte Triglyceride,
- alkoxilierte Fettsäurealkylester der Formel (E4-I)

   R²²CO-(OCH₂CHR²³)_{w}OR²⁴ (E4-I)

   in der R²²CO für einen linearen oder verzweigten, gesättigten und/oder ungesättigten Acylrest mit 6 bis 22 Kohlenstoffatomen, R²³ für Wasserstoff oder Methyl, R²⁴ für lineare oder verzweigte Alkylreste mit 1 bis 4 Kohlenstoffatomen und w für Zahlen von 1 bis 20 steht,
- Aminoxide,
- Hydroxymischether,
- Sorbitanfettsäureester und Anlagerungeprodukte von Ethylenoxid an Sorbitanfettsäureester wie beispielsweise die Polysorbate,
- Zuckerfettsäureester und Anlagerungsprodukte von Ethylenoxid an Zuckerfettsäureester,
- Anlagerungsprodukte von Ethylenoxid an Fettsäurealkanolamide und Fettamine,
- Zuckertenside vom Typ der Alkyl- und Alkenyloligoglykoside gemäß Formel (E4-II),

   R²⁵O-[G]p (E4-II)

   in der R²⁵ für einen Alkyl- oder Alkenylrest mit 4 bis 22 Kohlenstoffatomen, G für einen Zuckerrest mit 5 oder 6 Kohlenstoffatomen und p für Zahlen von 1 bis 10 steht. Sie können nach den einschlägigen Verfahren der präparativen organischen Chemie erhalten werden. Die Alkyl- und Alkenyloligoglykoside können sich von Aldosen bzw. Ketosen mit 5 oder 6 Kohlenstoffatomen, vorzugsweise von Glucose, ableiten. Die bevorzugten Alkyl- und/oder Alkenyloligoglykoside sind somit Alkyl- und/oder Alkenyloligoglucoside. Die Indexzahl p in der allgemeinen Formel (E4-II) gibt den Oligomerisierungsgrad (DP), d. h. die Verteilung von Mono- und Oligoglykosiden an und steht für eine Zahl zwischen 1 und 10. Während p im einzelnen Molekül stets ganzzahlig sein muß und hier vor allem die Werte p = 1 bis 6 annehmen kann, ist der Wert p für ein bestimmtes Alkyloligoglykosid eine analytisch ermittelte rechnerische Größe, die meistens eine gebrochene Zahl darstellt. Vorzugsweise werden Alkyl-und/oder Alkenyloligoglykoside mit einem mittleren Oligomerisierungsgrad p von 1,1 bis 3,0 eingesetzt. Aus anwendungstechnischer Sicht sind solche Alkyl- und/oder Alkenyloligoglykoside bevorzugt, deren Oligomerisierungsgrad kleiner als 1,7 ist und insbesondere zwischen 1,2 und 1,4 liegt. Der Alkyl- bzw. Alkenylrest R²⁵ kann sich von primären Alkoholen mit 4 bis 11, vorzugsweise 8 bis 10 Kohlenstoffatomen ableiten. Typische Beispiele sind Butanol, Capronalkohol, Caprylalkohol, Caprinalkohol und Undecylalkohol sowie deren technische Mischungen, wie sie beispielsweise bei der Hydrierung von technischen Fettsäuremethylestern oder im Verlauf der Hydrierung von Aldehyden aus der Roelen'schen Oxosynthese erhalten werden. Bevorzugt sind Alkyloligoglucoside der Kettenlänge C₈-C₁₀ (DP = 1 bis 3), die als Vorlauf bei der destillativen Auftrennung von technischem C₈-C₁₈-Kokosfettalkohol anfallen und mit einem Anteil von weniger als 6 Gew.-% C₁₂-Alkohol verunreinigt sein können sowie Alkyloligoglucoside auf Basis technischer C_{9/11}-Oxoalkohole (DP = 1 bis 3). Der Alkyl- bzw. Alkenylrest R²⁵ kann sich ferner auch von primären Alkoholen mit 12 bis 22, vorzugsweise 12 bis 14 Kohlenstoffatomen ableiten. Typische Beispiele sind Laurylalkohol, Myristylalkohol, Cetylalkohol, Palmoleylalkohol, Stearylalkohol, Isostearylalkohol, Oleylalkohol, Elaidylalkohol, Petroselinylalkohol, Arachylalkohol, Gadoleylalkohol, Behenylalkohol, Erucylalkohol, Brassidylalkohol sowie deren technische Gemische, die wie oben beschrieben erhalten werden können. Bevorzugt sind Alkyloligoglucoside auf Basis von gehärtetem C_{12/14}-Kokosalkohol mit einem DP von 1 bis 3.
- Zuckertenside vom Typ der Fettsäure-N-alkylpolyhydroxyalkylamide, ein nichtionisches Tensid der Formel (E4-III), 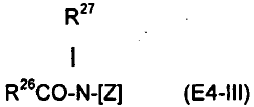 in der R²⁶CO für einen aliphatischen Acylrest mit 6 bis 22 Kohlenstoffatomen, R²⁷ für Wasserstoff, einen Alkyl- oder Hydroxyalkylrest mit 1 bis 4 Kohlenstoffatomen und [Z] für einen linearen oder verzweigten Polyhydroxyalkylrest mit 3 bis 12 Kohlenstoffatomen und 3 bis 10 Hydroxylgruppen steht. Bei den Fettsäure-N-alkylpolyhydroxyalkylamiden handelt es sich um bekannte Stoffe, die üblicherweise durch reduktive Aminierung eines reduzierenden Zuckers mit Ammoniak, einem Alkylamin oder einem Alkanolamin und nachfolgende Acylierung mit einer Fettsäure, einem Fettsäurealkylester oder einem Fettsäurechlorid erhalten werden können. Vorzugsweise leiten sich die Fettsäure-N-alkylpolyhydroxyalkylamide von reduzierenden Zuckern mit 5 oder 6 Kohlenstoffatomen, insbesondere von der Glucose ab. Die bevorzugten Fettsäure-N-alkylpolyhydroxyalkylamide stellen daher Fettsäure-N-atkylglucamide dar, wie sie durch die Formel (E4-IV) wiedergegeben werden:

   R²⁸CO-NR²⁹ -CH₂-(CHOH)₄CH₂OH (E4-IV)

Vorzugsweise werden als Fettsäure-N-alkylpolyhydroxyalkylamide Glucamide der Formel (E4-IV) eingesetzt, in der R²⁹ für Wasserstoff oder eine Alkylgruppe steht und R²⁸CO für den Acylrest der Capronsäure, Caprylsäure, Caprinsäure, Laurinsäure, Myristinsäure, Palmitinsäure, Palmoleinsäure, Stearinsäure, lsostearinsäure, Ölsäure, Elaidinsäure, Petroselinsäure, Linolsäure, Linolensäure, Arachinsäure, Gadoleinsäure, Behensäure oder Erucasäure bzw. technischer Mischungen dieser Säuren steht. Besonders bevorzugt sind Fettsäure-N-alkylglucamide der Formel (E4-IV), die durch reduktive Aminierung von Glucose mit Methylamin und anschließende Acylierung mit Laurinsäure oder C12/14-Kokosfettsäure bzw. einem entsprechenden Derivat erhalten werden. Weiterhin können sich die Polyhydroxyalkylamide auch von Maltose und Palatinose ableiten.

Als bevorzugte nichtionische Tenside haben sich die Alkylenoxid-Anlagerungsprodukte an gesättigte lineare Fettalkohole und Fettsäuren mit jeweils 2 bis 30 Mol Ethylenoxid pro Mol Fettalkohol bzw. Fettsäure erwiesen. Zubereitungen mit hervorragenden Eigenschaften werden ebenfalls erhalten, wenn sie als nichtionische Tenside Fettsäureester von ethoxyliertem Glycerin enthalten.

Diese Verbindungen sind durch die folgenden Parameter gekennzeichnet. Der Alkylrest enthält 6 bis 22 Kohlenstoffatome und kann sowohl linear als auch verzweigt sein. Bevorzugt sind primäre lineare und in 2-Stellung methylverzweigte aliphatische Reste. Solche Alkylreste sind beispielsweise 1-Octyl, 1-Decyl, 1-Lauryl, 1-Myristyl, 1-Cetyl und 1-Stearyl. Besonders bevorzugt sind 1-Octyl, 1-Decyl, 1-Lauryl, 1-Myristyl. Bei Verwendung sogenannter "Oxo-Alkohole" als Ausgangsstoffe überwiegen Verbindungen mit einer ungeraden Anzahl von Kohlenstoffatomen in der Alkylkette.

Weiterhin können als nichtionische Tenside die Zuckertenside enthalten sein. Diese sind bevorzugt in Mengen von 0,1 bis 20 Gew.%, bezogen auf die jeweilige gesamte Zusammensetzung, enthalten. Mengen von 0,5 bis 15 Gew.% sind besonders bevorzugt, und ganz besonders bevorzugt sind Mengen von 0,5 bis 7,5 Gew.%.

Bei den als Tensid eingesetzten Verbindungen mit Alkylgruppen kann es sich jeweils um einheitliche Substanzen handeln. Es ist jedoch in der Regel bevorzugt, bei der Herstellung dieser Stoffe von nativen pflanzlichen oder tierischen Rohstoffen auszugehen, so dass man Substanzgemische mit unterschiedlichen, vom jeweiligen Rohstoff abhängigen Alkylkettenlängen erhält.

Bei den Tensiden, die Anlagerungsprodukte von Ethylen- und/oder Propylenoxid an Fettalkohole oder Derivate dieser Anlagerungsprodukte darstellen, können sowohl Produkte mit einer "normalen" Homologenverteilung als auch solche mit einer eingeengten Homologenverteilung verwendet werden. Unter "normaler" Homologenverteilung werden dabei Mischungen von Homologen verstanden, die man bei der Umsetzung von Fettalkohol und Alkylenoxid unter Verwendung von Alkalimetallen, Alkalimetallhydroxiden oder Alkalimetallalkoholaten als Katalysatoren erhält. Eingeengte Homologenverteilungen werden dagegen erhalten, wenn beispielsweise Hydrotalcite, Erdalkalimetallsalze von Ethercarbonsäuren, Erdalkalimetalloxide, - hydroxide oder -alkoholate als Katalysatoren verwendet werden. Die Verwendung von Produkten mit eingeengter Homologenverteilung kann bevorzugt sein.

Die weiteren Tenside werden in der Regel in Mengen von 0,1 bis 45 Gew.%, bevorzugt 0,5 bis 30 Gew.% und ganz besonders bevorzugt von 0,5 bis 25 Gew.%, bezogen auf die jeweilige gesamte Zusammensetzung, eingesetzt. Dabei hängt die eingesetzte Menge wesentlich davon ab, welchen Zweck das erfindungsgemäße Mittel erfüllt. Handelt es sich um ein Shampoo oder ein anderes reinigendes Mittel, sind auch Tensidmengen über 45 Gew.% üblich.

Die Mittel können weiterhin mindestens einen Emulgator enthalten. Emulgatoren bewirken an der Phasengrenzfläche die Ausbildung von wasser- bzw. ölstabilen Adsorptionsschichten, welche die dispergierten Tröpfchen gegen Koaleszenz schützen und damit die Emulsion stabilisieren. Emulgatoren sind daher wie Tenside aus einem hydrophoben und einem hydrophilen Molekülteil aufgebaut. Hydrophile Emulgatoren bilden bevorzugt O/W- Emutsionen und hydrophobe Emulgatoren bilden bevorzugt W/O - Emulsionen. Die Auswahl dieser emulgierenden Tenside oder Emulgatoren richtet sich dabei nach den zu dispergierenden Stoffen und der jeweiligen äußeren Phase sowie der Feinteiligkeit der Emulsion. Erfindungsgemäß verwendbare Emulgatoren sind beispielsweise
- Anlagerungsprodukte von 4 bis 100 Mol Ethylenoxid und/oder 1 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe,
- C₁₂-C₂₂-Fettsäuremono- und -diester von Anlagerungsprodukten von 1 bis 30 Mol Ethylenoxid an Polyole mit 3 bis 6 Kohlenstoffatomen, insbesondere an Glycerin,
- Ethylenoxid- und Polyglycerin-Anlagerungsprodukte an Methylglucosid-Fettsäureester, Fettsäurealkanolamide und Fettsäureglucamide,
- C₈-C₂₂-Alkylmono- und -oligoglycoside und deren ethoxylierte Analoga, wobei Oligomerisierungsgrade von 1,1 bis 5, insbesondere 1,2 bis 2,0, und Glucose als Zuckerkomponente bevorzugt sind,
- Gemische aus Alkyl-(oligo)-glucosiden und Fettalkoholen zum Beispiel das im Handel erhältliche Produkt Montanov^{®}68,
- Anlagerungsprodukte von 5 bis 60 Mol Ethylenoxid an Rizinusöl und gehärtetes Rizinusöl,
- Partialester von Polyolen mit 3-6 Kohlenstoffatomen mit gesättigten Fettsäuren mit 8 bis 22 C-Atomen,
- Sterine. Als Sterine wird eine Gruppe von Steroiden verstanden, die am C-Atom 3 des Steroid-Gerüstes eine Hydroxylgruppe tragen und sowohl aus tierischem Gewebe (Zoosterine) wie auch aus pflanzlichen Fetten (Phytosterine) isoliert werden. Beispiele für Zoosterine sind das Cholesterin und das Lanosterin. Beispiele geeigneter Phytosterine sind Ergosterin, Stigmasterin und Sitosterin. Auch aus Pilzen und Hefen werden Sterine, die sogenannten Mykosterine, isoliert.
- Phospholipide. Hierunter werden vor allem die Glucose-Phospolipide, die z.B. als Lecithine bzw. Phospahtidylcholine aus z.B. Eidotter oder Pflanzensamen (z.B. Sojabohnen) gewonnen werden, verstanden.
- Fettsäureester von Zuckern und Zuckeralkoholen, wie Sorbit,
- Polyglycerine und Polyglycerinderivate wie beispielsweise Polyglycerinpoly-12-hydroxystearat (Handelsprodukt Dehymuls^{®} PGPH),
- lineare und verzweigte Fettsäuren mit 8 bis 30 C - Atomen und deren Na-, K-, Ammonium-, Ca-, Mg- und Zn - Salze.

Die Emulgatoren werden bevorzugt in Mengen von 0,1 bis 25 Gew.%, insbesondere 0,1 bis 3 Gew.%, bezogen auf die jeweilige gesamte Zusammensetzung, eingesetzt.

Eine andere bedeutende Gruppe von Weichspüler- und Wasch- und Reinigungsmittelinhaltsstoffen sind die Buildersubstanzen. Unter dieser Substanzklasse, werden sowohl organische als auch anorganische Gerüstsubstanzen verstanden. Es handelt sich dabei um Verbindungen, die sowohl eine Trägerfunktion in den erfindungsgemäßen Mitteln wahrnehmen können als auch bei der Anwendung als wasserenthärtende Substanz wirken.

Geeignete Builder sind beispielsweise Alkalimetallgluconate, -citrate, -nitrilotriacetate, -carbonate und -bicarbonate, insbesondere Natriumgluconat, -citrat und -nitrilotriacetat sowie Natrium- und Kaliumcarbonat und -bicarbonat, sowie Alkalimetall- und Erdalkalimetallhydroxide, insbesondere Natrium- und Kaliumhydroxid, Ammoniak und Amine, insbesondere Mono- und Triethanolamin, bzw. deren Mischungen. Hierzu zählen auch die Salze der Glutarsäure, Bernsteinsäure, Adipinsäure, Weinsäure und Benzolhexacarbonsäure sowie Phosphonate und Phosphate.

Brauchbare organische Gerüstsubstanzen sind beispielsweise die in Form ihrer Natriumsalze einsetzbaren Polycarbonsäuren, wobei unter Polycarbonsäuren solche Carbonsäuren verstanden werden, die mehr als eine Säurefunktion tragen. Beispielsweise sind dies Citronensäure, Adipinsäure, Bernsteinsäure, Glutarsäure, Äpfelsäure, Weinsäure, Maleinsäure, Fumarsäure, Zuckersäuren, Aminocarbonsäuren, Nitrilotriessigsäure (NTA), sofern ein derartiger Einsatz aus ökologischen Gründen nicht zu beanstanden ist, sowie Mischungen aus diesen. Bevorzugte Salze sind die Salze der Polycarbonsäuren wie Citronensäure, Adipinsäure, Bernsteinsäure, Glutarsäure, Weinsäure, Zuckersäuren und Mischungen aus diesen. Auch die Säuren an sich können eingesetzt werden. Die Säuren besitzen neben ihrer Builderwirkung typischerweise auch die Eigenschaft einer Säuerungskomponente und dienen somit, wie beispielsweise in den erfindungsgemäßen Granulaten, auch zur Einstellung eines niedrigeren und milderen pH-Wertes von Wasch- oder Reinigungsmitteln. Insbesondere sind hierbei Citronensäure, Bernsteinsäure, Glutarsäure, Adipinsäure, Gluconsäure und beliebige Mischungen aus diesen zu nennen.

Als Builder sind weiter polymere Polycarboxylate geeignet, dies sind beispielsweise die Alkalimetallsalze der Polyacrylsäure oder der Polymethacrylsäure, beispielsweise solche mit einer relativen Molekülmasse von 500 bis 70000 g/mol. Die (co-)polymeren Polycarboxylate können entweder als Pulver oder als wäßrige Lösung eingesetzt werden. Der Gehalt der Mittel an (co-) polymeren Polycarboxylaten beträgt vorzugsweise 0,5 bis 20 Gew.%, insbesondere 3 bis 10 Gew. %.

Zur Verbesserung der Wasserlöslichkeit können die Polymere auch Allylsulfonsäuren, Allyloxybenzolsulfonsäure und Methallylsulfonsäure, als Monomer enthalten. Insbesondere bevorzugt sind auch biologisch abbaubare Polymere aus mehr als zwei verschiedenen Monomereinheiten, beispielsweise solche, die als Monomere Salze der Acrylsäure und der Maleinsäure sowie Vinylalkohol bzw. Vinylalkohol-Derivate oder als Monomere Salze der Acrylsäure und der 2-Alkylallylsulfonsäure sowie Zucker-Derivate enthalten. Weitere bevorzugte Copolymere sind solche, die als Monomere vorzugsweise Acrolein und Acrylsäure/Acrylsäuresalze bzw. Acrolein und Vinylacetat aufweisen. Ebenso sind als weitere bevorzugte Buildersubstanzen polymere Aminodicarbonsäuren, deren Salze oder deren Vorläufersubstanzen zu nennen. Besonders bevorzugt sind Polyasparaginsäuren bzw. deren Salze und Derivate, die neben Co-builder-Eigenschaften auch eine bleichstabilisierende Wirkung aufweisen.

Weitere geeignete Buildersubstanzen sind Polyacetale, welche durch Umsetzung von Dialdehyden mit Polyolcarbonsäuren, welche 5 bis 7 C-Atome und mindestens 3 Hydroxylgruppen aufweisen, erhalten werden können. Bevorzugte Polyacetale werden aus Dialdehyden wie Glyoxal, Glutaraldehyd, Terephthalaldehyd sowie deren Gemischen und aus Polyolcarbonsäuren wie Gluconsäure und/oder Glucoheptonsäure erhalten.

Weitere geeignete organische Buildersubstanzen sind Dextrine, beispielsweise Oligomere bzw. Polymere von Kohlenhydraten, die durch partielle Hydrolyse von Stärken erhalten werden können. Die Hydrolyse kann nach üblichen, beispielsweise säure- oder enzymkatalysierten Verfahren durchgeführt werden. Vorzugsweise handelt es sich um Hydrolyseprodukte mit mittleren Molmassen im Bereich von 400 bis 500000 g/mol. Dabei ist ein Polysaccharid mit einem Dextrose-Äquivalent (DE) im Bereich von 0,5 bis 40, insbesondere von 2 bis 30 bevorzugt, wobei DE ein gebräuchliches Maß für die reduzierende Wirkung eines Polysaccharids im Vergleich zu Dextrose, welche ein DE von 100 besitzt, ist. Brauchbar sind sowohl Maltodextrine mit einem DE zwischen 3 und 20 und Trockenglucosesirupe mit einem DE zwischen 20 und 37 als auch sogenannte Gelbdextrine und Weißdextrine mit höheren Molmassen im Bereich von 2000 bits 30000 g/mol. Bei den oxidierten Derivaten derartiger Dextrine handelt es sich um deren Umsetzungsprodukte mit Oxidationsmitteln, welche in der Lage sind, mindestens eine Alkoholfunktion des Saccharidrings zur Carbonsäurefunktion zu oxidieren.

Auch Oxydisuccinate und andere Derivate von Disuccinaten, vorzugsweise Ethylendiamindisuccinat, sind weitere geeignete Co-builder. Dabei wird Ethylendiamin-N,N'-disuccinat (EDDS) bevorzugt in Form seiner Natrium- oder Magnesiumsalze verwendet: Weiterhin bevorzugt sind in diesem Zusammenhang auch Glycerindisuccinate und Glycerintrisuccinate. Geeignete Einsatzmengen liegen in zeolithhaltigen und/oder silicathaltigen Formulierungen bei 3 bis 15 Gew.-%.

Weitere brauchbare organische Co-builder sind beispielsweise acetylierte Hydroxycarbonsäuren bzw deren Salze, welche gegebenenfalls auch in Lactonform vorliegen können und welche mindestens 4 Kohlenstoffatome und mindestens eine Hydroxygruppe sowie maximal zwei Säuregruppen enthalten.

Eine weitere Substanzklasse mit Co-buildereigenschaften stellen die Phosphonate dar. Dabei handelt es sich insbesondere um Hydroxyalkan- bzw. Aminoalkanphosphonate. Unter den Hydroxyalkanphosphonaten ist das 1-Hydroxyethan-1,1-diphosphonat (HEDP) von besonderer Bedeutung als Co-builder. Es wird vorzugsweise als Natriumsalz eingesetzt, wobei das Dinatriumsalz neutral und das Tetranatriumsalz alkalisch (pH 9) reagiert. Als Aminoalkanphosphonate kommen vorzugsweise Ethylendiamintetramethylenphosphonat (EDTMP), Diethylentriaminpentamethylenphosphonat (DTPMP) sowie deren höhere Homologe in Frage. Sie werden vorzugsweise in Form der neutral reagierenden Natriumsalze, z.B. als Hexanatriumsalz der EDTMP bzw. als Hepta- und Octa-Natriumsalz der DTPMP, eingesetzt. Als Builder wird dabei aus der Klasse der Phosphonate bevorzugt HEDP verwendet. Die Aminoalkanphosphonate besitzen zudem ein ausgeprägtes Schwermetallbindevermögen. Dementsprechend kann es, insbesondere wenn die Mittel auch Bleiche enthalten, bevorzugt sein, Aminoalkanphosphonate, insbesondere DTPMP, einzusetzen, oder Mischungen aus den genannten Phosphonaten zu verwenden.

Darüber hinaus können alle Verbindungen, die in der Lage sind, Komplexe mit Erdalkaliionen auszubilden, als Co-builder eingesetzt werden.

Ein bevorzugt eingesetzter anorganischer Builder ist feinkristalliner, synthetischer und gebundenes Wasser enthaltender Zeolith. Der eingesetzte feinkristalline, synthetische und gebundenes Wasser enthaltende Zeolith ist vorzugsweise Zeolith A und/oder P. Als Zeolith P wird beispielsweise Zeolith MAP z.B. Doucil A24^{®} (Handelsprodukt der Firma Crosfield) eingesetzt. Geeignet sind jedoch auch Zeolith X sowie Mischungen aus A, X und/oder P, beispielsweise ein Co-Kristallisat aus den Zeolithen A und X, der Vegobond**^{®}** AX (Handelsprodukt der Condea Augusta S.p.A.). Der Zeolith kann als sprühgetrocknetes Pulver oder auch als ungetrocknete, von ihrer Herstellung noch feuchte, stabilisierte Suspension zum Einsatz kommen. Für den Fall, dass der Zeolith als Suspension eingesetzt wird, kann diese geringe Zusätze an nichtionischen Tensiden als Stabilisatoren enthalten, beispielsweise 1 bis 3 Gew.%, bezogen auf Zeolith, an ethoxylierten C₁₂-C₁₈-Fettalkoholen mit 2 bis 5 Ethylenoxidgruppen, C₁₂-C₁₄-Fettalkoholen mit 4 bis 5 Ethylenoxidgruppen oder ethoxylierten Isotridecanolen. Geeignete Zeolithe weisen eine mittlere Teilchengröße von weniger als 10 µm (Volumenverteilung; Meßmethode: Coulter Counter) auf und enthalten vorzugsweise 18 bis 22 Gew.-, insbesondere 20 bis 22 Gew.% an gebundenem Wasser. In bevorzugten Ausführungsformen sind Zeolithe in Mengen von 10 bis 94,5 Gew.% in dem Vorgemisch enthalten, wobei es kann besonders bevorzugt ist, wenn Zeolithe in Mengen von 20 bis 70, insbesondere 30 bis 60 Gew.% enthalten sind.

Geeignete Teilsubstitute für Zeolithe sind Schichtsilicate natürlichen und synthetischen Ursprungs. Ihre Verwendbarkeit ist nicht auf eine spezielle Zusammensetzung bzw. Strukturformel beschränkt. Bevorzugt sind hier jedoch Smectite, insbesondere Bentonite. Auch kristalline, schichtförmige Natriumsilicate der allgemeinen Formel NaMSiₓO_{2x+1 Y}H₂O, wobei M Natrium oder Wasserstoff bedeutet, x eine Zahl von 1,9 bis 4 und y eine Zahl von 0 bis 20 ist und bevorzugte Werte für x 2, 3 oder 4 sind, eigenen sich zur Substitution von Zeolithen oder Phosphaten. Bevorzugte kristalline Schichtsilicate der angegebenen Formel sind solche, in denen M für Natrium steht und x die Werte 2 oder 3 annimmt. Insbesondere sind sowohl β- als auch δ-Natriumdisilicate Na₂Si₂O₅ yH₂O bevorzugt.

Selbstverständlich ist auch ein Einsatz der allgemein bekannten Phosphate als Buildersubstanzen möglich, sofern ein derartiger Einsatz nicht aus ökologischen Gründen vermieden werden sollte. Geeignet sind insbesondere die Natriumsalze der Orthophosphate, der Pyrophosphate und insbesondere der Tripolyphosphate.

Die Mittel enthalten Builder bevorzugt in Mengen, bezogen auf die Zusammensetzung, von 0 bis 20 Gew.%, vorzugsweise 0,01 bis 12 Gew.%, insbesondere 0,1 bis 8 Gew.% äußerst bevorzugt 0,3 bis 5 Gew.%.

Neben den genannten Bestandteilen können die erfindungsgemäßen Wasch- und Reinigungsmittel zusätzlich einen oder mehrere Stoffe aus der Gruppe der Bleichmittel, Bleichaktivatoren, Enzyme, pH-Stellmittel, Fluoreszenzmittel, Farbstoffe, Schauminhibitoren, Silikonöle, Antiredepositionsmittel, optischen Aufheller, Vergrauungsinhibitoren, Farbübertragungsinhibitoren, Korrosionsinhibitoren und Silberschutzmittel enthalten. Diese Stoffe werden nachfolgend beschrieben.

Unter den als Bleichmittel dienenden, in Wasser H₂O₂ liefernden Verbindungen haben das Natriumperborattetrahydrat, das Natriumperboratmonohydrat und das Natriumpercarbonat besondere Bedeutung. Weitere brauchbare Bleichmittel sind beispielsweise Peroxypyrophosphate, Citratperhydrate sowie H₂O₂ liefernde persaure Salze oder Persäuren, wie Perbenzoate, Peroxophthalate, Diperazelainsäure, Phthaloiminopersäure oder Diperdodecandisäure. Auch beim Einsatz der Bleichmittel ist es möglich, auf den Einsatz von Tensiden und/oder Gerüststoffen zu verzichten, so dass reine Bleichmitteltabletten herstellbar sind. Sollen solche Bleichmitteltabletten zur Textilwäsche eingesetzt werden, ist eine Kombination von Natriumpercarbonat mit Natriumsesquicarbonat bevorzugt, unabhängig davon, welche weiteren Inhaltsstoffe in den Formkörpern enthalten sind. Werden Reinigungs- oder Bleichmitteltabletten für das maschinelle Geschirrspülen hergestellt, so können auch Bleichmittel aus der Gruppe der organischen Bleichmittel eingesetzt werden. Typische organische Bleichmittel sind die Diacylperoxide, wie z.B. Dibenzoylperoxid. Weitere typische organische Bleichmittel sind die Peroxysäuren, wobei als Beispiele besonders die Alkylperoxysäuren und die Arylperoxysäuren genannt werden. Bevorzugte Vertreter sind (a) die Peroxybenzoesäure und ihre ringsubstituierten Derivate, wie Alkylperoxybenzoesäuren, aber auch Peroxy-α-Naphtoesäure und Magnesium-monoperphthalat, (b) die aliphatischen oder substituiert aliphatischen Peroxysäuren, wie Peroxylaurinsäure, Peroxystearinsäure, ε-Phthalimidoperoxycapronsäure [Phthaloiminoperoxyhexansäure (PAP)], o-Carboxybenzamidoperoxycapronsäure, N-nonenylamidoperadipin-säure und N-nonenylamidopersuccinate, und (c) aliphatische und araliphatische Peroxydi-carbonsäuren, wie 1,12-Diperoxycarbonsäure, 1,9-Diperoxyazelainsäure, Diperocysebacinsäure, Diperoxybrassyisäure, die Diperoxyphthalsäuren, 2-Decyldiperoxybutan-1,4-disäure, N,N-Terephthaloyl-di(6-aminopercapronsäue) können eingesetzt werden.

Als Bleichmittel in Mitteln für das maschinelle Geschirrspülen können auch Chlor oder Brom freisetzende Substanzen eingesetzt werden. Unter den geeigneten Chlor oder Brom freisetzenden Materialien kommen beispielsweise heterocyclische N-Brom- und N-Chloramide, beispielsweise Trichlorisocyanursäure, Tribromisocyanursäure, Dibromisocyanursäure und/oder Dichlorisocyanursäure (DICA) und/oder deren Salze mit Kationen wie Kalium und Natrium in Betracht. Hydantoinverbindungen, wie 1,3-Dichlor-5,5-dimethylhydanthoin sind ebenfalls geeignet.

Um beim Waschen oder Reinigen bei Temperaturen von 60 °C und darunter eine verbesserte Bleichwirkung zu erreichen, können Bleichaktivatoren in die erfindungsgemäßen Wasch- und Reinigungsmittel eingearbeitet werden. Als Bleichaktivatoren können Verbindungen, die unter Perhydrolysebedingungen aliphatische Peroxocarbonsäuren mit vorzugsweise 1 bis 10 C-Atomen, insbesondere 2 bis 4 C-Atomen, und/oder gegebenenfalls substituierte Perbenzoesäure ergeben, eingesetzt werden. Geeignet sind Substanzen, die O- und/oder N-Acylgruppen der genannten C-Atomzahl und/oder gegebenenfalls substituierte Benzoylgruppen tragen. Bevorzugt sind mehrfach acylierte Alkylendiamine, insbesondere Tetraacetylethylendiamin (TAED), acylierte Triazinderivate, insbesondere 1,5-Diacetyl-2,4-dioxohexahydro-1,3,5-triazin (DADHT), acylierte Glykolurile, insbesondere Tetraacetylglykoluril (TAGU), N-Acylimide, insbesondere N-Nonanoylsuccinimid (NOSI), acylierte Phenolsulfonate, insbesondere n-Nonanoyl- oder Isononanoyloxybenzolsulfonat (n- bzw. iso-NOBS), Carbonsäureanhydride, insbesondere Phthalsäureanhydrid, acylierte mehrwertige Alkohole, insbesondere Triacetin, Ethylenglykoldiacetat und 2,5-Diacetoxy-2,5-dihydrofuran.

Zusätzlich zu den konventionellen Bleichaktivatoren oder an deren Stelle können auch sogenannte Bleichkatalysatoren enthalten sein. Bei diesen Stoffen handelt es sich um bleichverstärkende Übergangsmetallsalze bzw. Übergangsmetallkomplexe wie beispielsweise Mn-, Fe-, Co-, Ru- oder Mo-Salenkomplexe oder -Carbonylkomplexe. Auch Mn-, Fe-, Co-, Ru-, Mo-, Ti-, V- und Cu-Komplexe mit N-haltigen Tripod-Liganden sowie Co-, Fe-, Cu- und Ru-Amminkomplexe sind als Bleichkatalysatoren verwendbar.

Als Enzyme kommen solche aus der Klasse der Proteasen, Lipasen, Amylasen, Cellulasen bzw. deren Gemische in Frage. Besonders gut geeignet sind aus Bakterienstämmen oder Pilzen, wie Bacillus subtilis, Bacillus licheniformis und Streptomyces griseus gewonnene enzymatische Wirkstoffe. Vorzugsweise werden Proteasen vom Subtilisin-Typ und insbesondere Proteasen, die aus Bacillus lentus gewonnen werden, eingesetzt. Dabei sind Enzymmischungen, beispielsweise aus Protease und Amylase oder Protease und Lipase oder Protease und Cellulase oder aus Cellulase und Lipase oder aus Protease, Amylase und Lipase oder Protease, Lipase und Cellulase, insbesondere jedoch Cellulase-haltige Mischungen von besonderem Interesse. Auch Peroxidasen oder Oxidasen haben sich in einigen Fällen als geeignet erwiesen. Die Enzyme können an Trägerstoffen adsorbiert und/oder in Hüllsubstanzen eingebettet sein, um sie gegen vorzeitige Zersetzung zu schützen. Der Anteil der Enzyme, Enzymmischungen oder Enzymgranulate in den erfindungsgemäßen Formkörpern kann beispielsweise etwa 0,1 bis 5 Gew.%, vorzugsweise 0,1 bis etwa 2 Gew.% betragen. Zu den am häufigsten verwendeten Enzymen gehören Lipasen, Amylasen, Cellulasen und Proteasen. Bevorzugte Proteasen sind z.B. BLAP®140 der Fa. Biozym, Optimase®-M-440 und Opticlean®-M-250 der Fa. Solvay Enzymes; Maxacal®CX und Maxapem® oder Esperase® der Fa. Gist Brocades oder auch Savinase® der Fa. Novo. Besonders geeignete Cellulasen und Lipasen sind Celluzym® 0,7 T und Lipolase® 30 T der Fa. Novo Nordisk. Besondere Verwendung als Amylasen finden Duramyl® und Termamyl® 60 T, und Termamyl® 90 T der Fa. Novo, Amylase-LT® der Fa. Solvay Enzymes oder Maxamyl® P5000 der Fa. Gist Brocades. Auch andere Enzyme können verwendet werden.

Zusätzlich können die Wasch- und Reinigungsmittel auch Komponenten enthalten, welche die Ölund Fettauswaschbarkeit aus Textilien positiv beeinflussen (sogenannte soil repellents). Dieser Effekt wird besonders deutlich, wenn ein Textil verschmutzt wird, das bereits vorher mehrfach mit einem erfindungsgemäßen Waschmittel, das diese öl- und fettlösende Komponente enthält, gewaschen wurde. Zu den bevorzugten öl- und fettlösenden Komponenten zählen beispielsweise nichtionische Celluloseether wie Methylcellulose und Methylhydroxy-propylcellulose mit einem Anteil an Methoxyl-Gruppen von 15 bis 30 Gew.% und an Hydroxypropoxyl-Gruppen von 1 bis 15 Gew.-%, jeweils bezogen auf den nichtionischen Celluloseether, sowie die aus dem Stand der Technik bekannten Polymere der Phthalsäure und/oder der Terephthalsäure bzw. von deren Derivaten, insbesondere Polymere aus Ethylenterephthalaten und/oder Polyethylenglykolterephthalaten oder anionisch und/oder nichtionisch modifizierten Derivaten von diesen. Besonders bevorzugt von diesen sind die sulfonierten Derivate der Phthalsäure- und der Terephthalsäure-Polymere.

Weiterhin können die Mittel als optische Aufheller Derivate der Diaminostilbendisulfonsäure bzw. deren Alkalimetallsalze enthalten. Geeignet sind z.B. Salze der 4,4'-Bis(2-anilino-4-morpholino-1,3,5-triazinyl-6-amino)stilben-2,2'-disulfonsäure oder gleichartig aufgebaute Verbindungen, die anstelle der Morpholino-Gruppe eine Diethanolaminogruppe, eine Methylaminogruppe, eine Anilinogruppe oder eine 2-Methoxyethylaminogruppe tragen. Weiterhin können Aufheller vom Typ der substituierten Diphenylstyryle anwesend sein, z.B. die Alkalisalze des 4,4'-Bis(2-sulfostyryl)di-phenyls, 4,4'-Bis(4-chlor-3-sulfostyryl)diphenyls, oder 4-(4-Chlorstyryl)-4'-(2-sulfostyryl)diphenyls. Auch Gemische der vorgenannten Aufheller können verwendet werden.

Um den ästhetischen Eindruck der erfindungsgemäßen Mittel zu verbessern, können sie mit geeigneten Farbstoffen eingefärbt werden. Bevorzugte Farbstoffe, deren Auswahl dem Fachmann keinerlei Schwierigkeit bereitet, besitzen eine hohe Lagerstabilität und Unempfindlichkeit gegenüber den übrigen Inhaltsstoffen der Mittel und gegen Licht sowie keine ausgeprägte Substantivität gegenüber Textilfasern, um diese nicht anzufärben.

Erfindungsgemäß sind. Wasch- und Reinigungsmittel auch Geschirrspülmittel. Die erfindungsgemäßen Geschirrspülmittel können zum Schutze des Spülgutes oder der Maschine Korrosionsinhibitoren enthalten, wobei besonders Silberschutzmittel im Bereich des maschinellen Geschirrspülens eine besondere Bedeutung haben. Allgemein können vor allem Silberschutzmittel ausgewählt aus der Gruppe der Triazole, der Benzotriazole, der Bisbenzotriazole, der Aminotriazole, der Alkylaminotriazole und der Übergangsmetallsalze oder -komplexe eingesetzt werden. Besonders bevorzugt zu verwenden sind Benzotriazol und/oder Alkylaminotriazol. Man findet in Reinigerformulierungen darüber hinaus häufig aktivchlorhaltige Mittel, die das Korrodieren der Silberoberfläche deutlich vermindern können. In chlorfreien Reinigern werden besonders sauerstoff- und stickstoffhaltige organische redoxaktive Verbindungen, wie zwei- und dreiwertige Phenole, z.B. Hydrochinon, Brenzkatechin, Hydroxyhydrochinon, Gallussäure, Phloroglucin, Pyrogallol bzw. Derivate dieser Verbindungsklassen. Auch salz- und komplexartige anorganische Verbindungen, wie Salze der Metalle Mn, Ti, Zr, Hf, V, Co und Ce finden häufig Verwendung. Bevorzugt sind hierbei die Übergangsmetallsalze, die ausgewählt sind aus der Gruppe der Mangan und/oder Cobaltsalze und/oder -komplexe, besonders bevorzugt der Cobalt(ammin)-Komplexe, der Cobalt(acetat)-Komplexe, der Cobalt-(Carbonyl)-Komplexe, der Chloride des Cobalts oder Mangans und des Mangansulfats. Ebenfalls können Zinkverbindungen zur Verhinderung der Korrosion am Spülgut eingesetzt werden.

Besondere Inhaltsstoffe, die in erfindungsgemäßen Mitteln für das maschinelle Geschirrspülen oder die Reinigung harter Oberflächen genutzt werden können, sind Substanzen, die das Wiederanschmutzen von Oberflächen verhindern und/oder die Schmutzablösung nach einmaliger Anwendung erleichtern (sogenannte "Soil-Release-Verbindungen").

Zu den verwendbaren Soil-Release Verbindungen zählen alle im Stand der Technik bekannten Verbindungen. Besonders geeignet sind kationische Polymere, wie beispielsweise Hydroxypropyltrimethylammonium-Guar; Copolymere von Aminoethylmethacrylat und Acrylamid sowie Copolymere von Dimethyldiallylammoniumchlorid und Acrylamid, Polymere mit IminoGruppen, kationische Cellulosederivate, kationische Homo- und/oder Copolymere (Monomereinheiten: quaternisierte Ammoniumalkylmethacrylatgruppen).

Besonders bevorzugt sind die kationischen Polymeren ausgewählt aus kationischen Polymerisaten von Copolymeren von Monomeren wie Trialkylammoniumalkyl(meth)acrylat bzw. -acrylamid; Dialkyldiallyldiammoniumsalze; polymeranalogen Umsetzungsprodukten von Ethern oder Estern von Polysacchariden mit Ammoniumseitengruppen, insbesondere Guar-, Cellulose- und Stärkederivate; Polyaddukte von Ethylenoxid mit Ammoniumgruppen; quaternäre Ethyleniminpolymere und Polyester und Polyamide mit quaternären Seitengruppen als Soil-Release-Verbindungen. Außergewöhnlich bevorzugt im Rahmen dieser Anmeldung sind auch natürliche Polyuronsäuren und verwandte Substanzen, sowie Polyampholyte und hydrophobierte Polyampholyte, bzw. Gemische dieser Substanzen.

Diese Aufzählung von Weichspüler- und Wasch- und Reinigungsmittelinhaltsstoffen ist keineswegs abschließend, sondern gibt lediglich die wesentlichsten typischen Inhaltsstoffe derartiger Mittel wieder. Insbesondere können, soweit es sich um flüssige oder gelförmige Zubereitungen handelt, in den Mitteln auch organische Lösungsmittel enthalten sein. Vorzugsweise handelt es sich um einoder mehrwertige Alkohole mit 1 bis 4 C-Atomen. Bevorzugte Alkohole in solchen Mitteln sind Ethanol, 1,2-Propandio, Glycerin sowie Gemische aus diesen Alkoholen. In bevorzugten Ausführungsformen enthalten derartige Mittel 2 bis 12 Gew.% solcher Alkohole.

Grundsätzlich können die Mittel verschiedene Aggregatszustände aufweisen.
In einer weiteren bevorzugten Ausführungsform handelt es sich bei den Weichspülern, Wasch-oder Reinigungsmitteln um flüssige oder gelförmige Mittel, insbesondere um Flüssigwaschmittel oder flüssige Geschirrspülmittel oder Reinigungsgele, wobei es sich insbesondere auch um gelförmige Reinigungsmittel für Spültoiletten handeln kann.

Es handelt sich dabei vorzugsweise um gelförmige, strukturviskose Reinlgungsmittel mit einer Viskosität von 30000 - 150000 mPas, das als Gelbildner ein Polysaccharid, als Emulgator und netzaktive Komponente ein C₈₋₁₁₀-Alkylpolyglycosid odar C₁₂₋₁₄-Alkylpolyglycosid und Parfümöl enthalten. Als zusätzliche Co-Tenside können Fettalkoholethersutfate (FAEOS) und Fettalkoholsulfate (FAS) enthalten sein. Das Verhältnis APG zu Co-Tensid ist dann In der Regel größer als 1, vorzugsweise liegt es zwischen 50:1 und 1:1, besonders bevorzugt zwischen 10:1 und 1.5 zu 1 und ganz besonders bevorzugt zwischen 5:1 und 1,8:1. Insbesondere handelt es sich dabei um stabile, gelförmige, scherverdünnende Reinigungsmittel enthaltend Polysaccharid, ein Tensidsystem und Parfumkompanenten, die dadurch gekennzeichnet sind, dass
- ein Polysaccharid, bevorzugt ein Xanthan-Gum, in Mengen zwischen 1 und 5 Gew.% bevorzugt 1 bis 4 Gew.%, besonders bevorzugt 1,5 bis 3,5 Gew.% und ganz besonders bevorzugt 1,8 bis 3 Gew.%,
- als eine Komponente des Tensidsystems ein C₈₋₂₂₋Alkylpolyglycosid in Mengen zwischen 3 und 25 Gew.% bevorzugt 4 und 20 Gew.-% besonders bevorzugt 5 und 15 Gew.% und ganz besonders bevorzugt 5 und 12 Gew.% und
- die Parfumkomponente oder die Parfumkornponenten bis 15 Gew.% bevorzugt In 2 bis 12 Gew.% besonders bevorzugt in 3 bis 8 Gew.%
- sowie ggf weitere inhaltsstoffe wie kalklösende Mittel, Farbstoffe, keimhemmende Mittel (beispielsweise Isothiazoüngemische, Natriumbenzoat oder Salicylsäure), Perlglanzmittel, Stabilisatoren Reinigungsverstarker und Geruchsabsorber enthalten sind
- und die Mittel eine Viskosität von 30000 bis 150000 mPas aufweisen, gemessen mit einem Brookflied Rotationsviskosimeter, Typ RVT mit Helipath-Einrichtung und der Spindel TA bei 1 U/min und 23 °C.

Weitere typische Reinigungsmittel, die die erfindungsgemäßen Pro-Fragrances enthalten können, sind flüssigen oder gelförmige Reiniger für harte Oberflächen, insbesondere sogenannte Allzweckreinlger, Glaeselniger, Boden- oder Badezimmerreiniger sowie spezielle Ausführungeformen derartiger Reiniger wozu saure oder alkallsche Formen von Allzweckreinigern ebenso wie Glasreiniger mit sogenannter Anti-Rain-Wirkung gehören. Dabei können diese flüssigen Reinigungsmittel sowohl in einer als auch In mehreren Phasen vorliegen, In einer insbesondere bevorzugten Ausführungsform weisen die Reiniger 2 verschiedene Phasen auf.

Reiniger ist dabei im weitesten Sinne eine Bezeichnung für - zumeist Tensid-haltige - Formulierungen mit sehr weitem Einsatzbereich und davon abhängig sehr unterschiedlicher Zusammensetzung. Die wichtigsten Marktsegmente sind Haushalts-Reiniger, industrielle (technische) und institutionelle Reiniger. Nach dem pH-Wert unterscheidet man alkalische, neutrale und saure Reiniger, nach der Angebotsform flüssige und feste Reiniger (auch in Tablettenform). Die sogenannten Reiniger für harte Oberflächen sollen im Unterschied etwa zu Geschirrspülmitteln, die ebenfalls mit in die Produktgruppe der Reiniger eingeordnet werden, sowohl im konzentrierten Zustand als auch in verdünnter wäßriger Lösung. in Verbindung mit mechanischer Energie ein optimales Anwendungsprofil zeigen. Kaltreiniger entfalten ihre Leistung ohne erhöhte Temperatur. Maßgebend für die Reinigungswirkung sind v.a. Tenside und/oder Alkaliträger, alternativ Säuren, ggf. auch Lösungsmittel wie Glykolether und niedere Alkohole. Im Allgemeinen sind in den Formulierungen darüber hinaus Builder, je nach Reiniger-Typ auch Bleichmittel, Enzyme, keimmindernde oder desinfizierende Zusätze sowie Parfümöle und Farbstoffe enthalten. Reiniger können auch als Mikroemulsionen formuliert sein. Der Reinigungserfolg hängt in hohem Maße von der- auch geographisch sehr unterschiedlichen - Schmutzart und den Eigenschaften der zu reinigenden Oberflächen ab.

Haushalts-Reiniger: können als Universal-Reiniger oder als Spezial-Reiniger für u.a. Keramik, Fliesen, Fenster, Kunststoffe, (Teppich-)Böden, Kochfelder, Backöfen, Mikrowellenherde, als Sanitär-Reiniger oder WC-Reiniger formuliert werden. Rohr-Reiniger sind alkalisch eingestellt und bestehen z.B. aus festem Natriumhydroxid und Aluminium-Pulver, bei dessen Auflösung der entstehende Wasserstoff für eine entsprechende Verwirbelung in den freizuspülenden Rohrsegmenten sorgt. Sanitär-Reiniger enthalten neben Tensid und Builder v.a. keimmindernde Wirkstoffe, wobei das früher verwendete Natriumhypochlorit teilweise durch Wasserstoffperoxid oder andere Persauerstoff-Verbindungen ersetzt ist. WC-Reiniger sind überwiegend sauer, manchmal auch alkalisch eingestellt, wobei im ersteren Fall die ursprünglich verwendete Phosphorsäure und das Natriumhydrogensulfat weitgehend durch organische Säuren, v.a. Citronensäure, ersetzt sind. Zu den Spezial-Reinigern. gehören im Do-it-yourself-Bereich auch Kfz-, Autoscheiben-, Felgen-, Motoren- und Farbauftragsgeräte-Reiniger.

Neben den genannten Komponenten können die erfindungsgemäßen Mittel weitere Hilfs- und Zusatzstoffe enthalten, wie sie in derartigen Mitteln üblich sind. Hierzu zählen insbesondere Polymere, Soil-Release-Wirkstoffe, Lösungsmittel (z.B. Ethanol, Isopropanol, Glykolether), Lösungsvermittler, Hydrotrope (z.B. Cumolsulfonat, Octylsulfat, Butylglucosid, Butylglykol), Reinigungsverstärker, Viskositätsregler (z.B. synthetische Polymere wie Polysaccharide, Polyacrylate, in der Natur vorkommenden Polymere und deren Derivate wie Xanthangum, weitere Polysaccharide und/oder Gelatine), pH-Regulatoren (z.B. Citronensäure, Alkanolamine oder NaOH), Desinfektionsmittel, Antistatika, Konservierungsmittel, Bleichsysteme, Enzyme, Farbstoffe sowie Trübungsmittel oder auch Hautschutzmittel.

Die Menge an derartigen Zusätzen liegt üblicherweise nicht über 12 Gew.% im Reinigungsmittel. Die Untergrenze des Einsatzes hängt von der Art des Zusatzstoffes ab und kann beispielsweise bei Farbstoffen bis zu 0,001 Gew.% und darunter betragen. Vorzugsweise liegt die Menge an Hilfsstoffen zwischen 0,01 und 7 Gew.%, insbesondere 0,1 und 4 Gew.%.

Die genannten Mittel können weiterhin Bindemittel enthalten, die allein oder in Mischung mit anderen Bindemitteln eingesetzt werden können. Bevorzugte Bindemittel sind Polyethylenglykole, 1.,2-Polypropylenglykole sowie modifizierte Polyethylenglykole und Polypropylenglykole. Zu den modifizierten Polyalkylenglykolen zählen insbesondere die Sulfate und/oder die Disulfate von Polyethylenglykolen oder Polypropylenglykolen mit einer relativen Molekülmasse zwischen 600 und 12000 und insbesondere zwischen 1000 und 4000. Eine weitere Gruppe besteht aus Mono-und/oder Disuccinaten der Polyalkylenglykole, welche wiederum relative Molekülmassen zwischen 600 und 6000, vorzugsweise zwischen 1000 und 4000 aufweisen.

Im Rahmen dieser Erfindung zählen zu Polyethylenglykolen solche Polymere, bei deren Herstellung neben Ethylenglykol ebenso C₃-C₅-Glykole sowie Glycerin und Mischungen aus diesen als Startmoleküle eingesetzt werden. Ferner werden auch ethoxylierte Derivate wie Trimethylolpropan mit 5 bis 30 Ethylenoxid (EO) umfasst. Die vorzugsweise eingesetzten Polyethylenglykole können eine lineare oder verzweigte Struktur aufweisen, wobei insbesondere lineare Polyethylenglykole bevorzugt sind. Zu den insbesondere bevorzugten Polyethylenglykolen gehören solche mit relativen Molekülmassen zwischen 2000 und 12000, vorteilhafterweise um 4000, wobei Polyethylen-glykole mit relativen Molekülmassen unterhalb 3500 und oberhalb 5000 insbesondere in Kombination mit Polyethylenglykolen mit einer relativen Molekülmasse um 4000 eingesetzt werden können und derartige Kombinationen vorteilhafterweise zu mehr als 50 Gew.%, bezogen auf die gesamte Menge der Polyethylenglykole, Polyethylenglykole mit einer relativen Molekülmasse zwischen 3500 und 5000 aufweisen. Als Bindemittel können jedoch auch Polyethylenglykole eingesetzt werden, welche an sich bei Raumtemperatur und einem Druck von 1 bar in flüssigem Stand vorliegen; hier ist vor allem von Polyethylenglykol mit einer relativen Molekülmasse von 200, 400 und 600 die Rede. Allerdings sollten diese an sich flüssigen Polyethylenglykole nur in einer Mischung mit mindestens einem weiteren Bindemittel eingesetzt werden, wobei diese Mischung wieder den erfindungsgemäßen Anforderungen genügen muss, also einen Schmelzpunkt bzw. Erweichungspunkt von mindestens oberhalb 45 °C aufweisen muss.

Ebenso eignen sich als Bindemittel niedermolekulare Polyvinylpyrrolidone und Derivate von diesen mit relativen Molekülmassen bis maximal 30000. Bevorzugt sind hierbei relative Molekülmassenbereiche zwischen 3000 und 30000, beispielsweise um 10000. Polyvinylpyrrolidone werden vorzugsweise nicht als alleinige Bindemittel, sondern in Kombination mit anderen, insbesondere in Kombination mit Polyethylenglykolen, eingesetzt.

Als geeignete und altbekannte Desintegrationshilfsmittel können die erfindungsgemaßen Mittel beispielsweise Carbonat/Citronensäure-Systeme enthalten, wobei auch andere organische Säuren eingesetzt werden können. Quellende Desintegrationshilfsmittel sind beispielsweise synthetische Polymere wie Polyvlnylpyrrolidon (PVP) oder natürliche Polymere bzw modifizierte Naturstoffe wie Cellulose und Stärke und ihre Derivate, Alginate oder Caseln-Derivate,

Als bevorzugte Desintegrationsmittel werden im Rahmen der vorliegenden Erfindung Desintegrationsmittel auf Cellulosebasis eingesetzt, 50 dass bevorzugte Wasch- und Reinigungsmittelformkörper ein solches Desintegrationsmittel auf Cellulosebasis in Mengen von 0,5 bis 10 Gew.%, vorzugsweise 3 bis 7 Gew.% und insbesondere 4 bis 6 Gew.-% enthalten.

In einer bevorzugten Variante enthalten die Wasch- und Reinigungsmittel, insbesondere in Form von Formkörper wie Tabletten 0.5 bis 10 Gew.%, vorzugsweise 3 bis 7 Gew.% und Insbesondere 4 bis 6 Gew.% eines oder mehrerer Desintegrationshilfsmittel, jeweils bezogen auf das Formkörpergewicht.

Ein weiterer Gegenstand der vorllegenden Erfindung sind Kosmetika (kosmetische Mittel) zur Haar- oder Hautbehandlung, enthaltend die erfindungsgemäßen Pro-Fragrances.
Vorzugsweise enlhalten diese Kosmetika (kosmetischen Mittel) die erfindungsgemäßen ProFragrances in Mengen von 0,001 bis 10 Gew.%, vorzugsweise von 0,01 bis 5 Gew.%, besonders bevorzugl von 0.02 bis 3 und insbesondere in Mengen von 0,05 bis 2 Gew.%, jeweils bezogen auf die Gesamtzusarnmensetzung des kosmetischen Mittels.

Die Gesamtmenge der Duftstoffe in den kosmetalchen Mittel beträgt dagegen vorzugsweise zwischen 0,01 und 5 Gew.%, besonders vorzugswelse zwischen 0,1 und 3 Gew.% sowie ganz besonders bevorzugt zwischen 0.5 und 2 Gew.% bezogen auf die Gesamtmenge des Mittels. Bevorzugt werden Mischungen verschiedener Duftstoffe (aus den verschiedenen oben genannten Duftstoffklassen) verwendet, die gemeinsam eine ansprechende Duftnote erzeugen. In diesem Fall ist die Gesamtmenge des mindestens einen Duftstoffs die Menge aller Duftsloffe in der Mischung zusammen bezogen auf die Gesamtmenge des Mittels.

In einer bevorzugten Ausführungsform handelt es sich bei den kosmetischen Mitteln um wässrige Zubereitungen, die oberflächenakilve Wirkstoffe enthalten und die sich Insbesondere zur Behandlung von Keratinfasern, insbesondere menschlichen Haaren, oder zur Behandlung von Haut eignen.

Bei den angesprochenen Haarbehandlungsmitteln handelt es sich dabei insbesondere um Mittel zur Behandlung von menschlichem Kopfhaar. Die gebräuchlichsten Mittel dieser Kategorie lassen sich einteilen in Haarwaschmittel, Haarpflegemittel, Haarverfestigungs- und Haarverformungsmittel sowie Haarfärbemittel und Haarentfernungsmittel. Zu den erfindungsgemäß bevorzugten, oberflächenaktive Wirkstoffe enthaltenden Mittel zählen insbesondere Haarwasch- und Pflegemittel. Ein derartiges Haarwaschmittel oder Haarshampoo besteht aus 10 bis 20, in Einzelfällen bis zu 30 Rezepturbestandteilen. Diese wässrigen Zubereitungen liegen meist in flüssig bis pastöser Form vor.
Die erfindungsgemäßen Kosmetika (kosmetischen Mittel), enthalten in der Regel noch weitere Inhaltsstoffe, die für diese Mittel üblich sind.

Vorzugsweise enthalten die erfindungsgemäßen kosmetischen Mittel oberflächenaktiven Wirkstoffe oder Waschaktivstoffe als weitere Inhaltsstoffe. Bevorzugt werden hierbei Fettalkoholpolyglykolethersulfate (Ethersulfate, Alkylethersulfate) eingesetzt, zum Teil in Kombination mit anderen meist anionischen Tensiden.
Neben den Alkylethersulfaten können bevorzugte Mittel zusätzlich weitere Tenside, wie Alkylsulfate, Alkylethercarboxylate, vorzugsweise mit Ethoxylierungsgraden von 4 bis 10, sowie tensidische Eiweiß-Fettsäure-Kondensate enthalten. Hier ist insbesondere das Eiweiß-Abitinsäure-Kondensat zu erwähnen. Auch Sulfobernsteinsäureester, Amidopropyplbetaine, Amphoacetate und Amphodiacetate sowie Alkylpolyglykoside sind in Haarshampoos bevorzugt eingesetzte Tenside.

Eine weitere Gruppe von Inhaltsstoffen wird unter dem Begriff Hilfsstoffe zusammengefasst und ist sehr vielfältig: z.B. erhöhen Zusätze von nichtionischen Tensiden wie ethoxylierten Sorbitanestern oder von Eiweiß-Hydrolysaten die Verträglichkeit bzw. wirken reizmindernd, z.B. in Baby-Shampoos; als Rückfetter zur Vorbeugung zu starker Entfettung bei der Haarwäsche dienen z.B. natürliche Öle oder synthetische Fettsäureester; als Feuchthaltemittel dienen Glycerin, Sorbit, Propylenglykol (s. Propandiole), Polyethylenglykole u.a. Polyole. Zur Verbesserung der Naßkämmbarkeit und Verminderung elektrostatischer Aufladung der Haare nach dem Trocknen können den Shampoos Kationtenside wie z.B. quartäre Ammonium-Verbindungen zugesetzt werden. Für ein farbiges, brillantes Erscheinungsbild werden Farbstoffe bzw. Perlglanzpigmente zugesetzt. Zur Einstellung der gewünschten Viskosität können Verdickungsmittel verschiedener Stoffklassen verwendet werden, eine pH-Stabilität wird durch Puffer z.B. auf der Basis von Citrat, Lactat oder Phosphat erzielt. Um eine ausreichende Haltbarkeit und Lagerfähigkeit zu gewährleisten, werden Konservierungsmittel wie z.B. 4-Hydroxybenzoesäureester zugesetzt; oxidationsempfindliche Inhaltsstoffe können durch Zusatz von Antioxidantien wie Ascorbinsäure, Butylmethoxyphenol oder Tocopherol geschützt werden.

Eine weitere bevorzugte Gruppe von inhaltsstoffen bilden spezielle Wirkstoffe für Spezial-Shampoos, z.B. Öle, Kräuterextrakte, Proteine, Vitamine und Lecithine in Shampoos für schnell fettendes, für besonders trockenes, für strapaziertes oder geschädigtes Haar. Wirkstoffe in Shampoos zur Bekämpfung von Schuppen haben meist eine breite wachstumshemmende Wirkung gegen Pilze und Bakterien. Insbesondere die fungistatischen Eigenschaften z.B. von Pyrithion-Salzen konnten als Ursache guter Antischuppen-Wirkung nachgewiesen werden. Zur Erzeugung einer angenehmen Duftnote enthalten die Haarshampoos Parfümöle. Dabei können die Shampoos ausschließlich die erfindungsgemäßen Kieselsäureester enthalten, es ist jedoch ebenfalls bevorzugt wenn die Haarshampoos nicht nur diese, sondern auch andere Duftstoffe daneben enthalten. Dabei können alle Üblichen, und in Haarshampoos zugelassenen Duftstoffe eingesetzt werden.

Haarpflegemittel haben zum Ziel den Naturzustand des frisch nachgewachsenen Haares möglichst lange zu erhalten und bei Schädigung wiederherzustellen. Merkmale die diesen Naturzustand charakterisieren sind seidiger Glanz, geringe Porosität, spannkräftige und dabei weiche Fülle und angenehm glattes Gefühl. Eine wichtige Voraussetzung hierfür ist eine saubere, schuppenfreie und nicht überfettete Kopfhaut. Zu den Haarpflegemitteln zählt man heute eine Vielzahl verschiedener Produkte, deren wichtigste Vertreter als Vorbehandlungsmittel, Haarwässer, Frisierhilsmittel, Haarspülungen und Kurpackungen bezeichnet werden, und deren Zusammensetzung wie bei den Haarwaschmitteln grob in Grundstoffe, Hilfsstoffe und spezielle Wirkstoffe aufgegliedert wird.

Als Grundstoffe dienen Fettalkohole, v.a. Cetylalkohol (1-Hexadecanol) und Stearylalkohol (1-Octadecanol), Wachse wie Bienenwachs, Wollwachs (Lanolin), Walrat und synthetische Wachse, Paraffine, Vaseline, Paraffinöl sowie als Lösungsmittel. v.a. Ethanol, 2-Propanol und Wasser. Hilfsstoffe sind Emulgatoren, Verdickungsmittel, Konservierungsmittel, Antioxidantien, Farbstoffe und Parfüm-Öle. Die heute wichtigste Gruppe spezieller Wirkstoffe in den Haarpflegemitteln sind die quartären Ammonium-Verbindungen. Man unterscheidet monomere (z.B.: Alkyltrimethylammoniumhalogenid mit v.a. der Lauryl-, Cetyl- oder Stearyl-Gruppe als Alkyl-Rest) und polymere quartäre Ammonium-Verbindungen [z.B.: quartäre Celluloseether-Derivate oder Poly(N,N-dimethyl-3,4-methylenpyrrolidiniumchlorid)]. Ihre Wirkung in Haarpflegemitteln beruht darauf, daß die positive Ladung der Stickstoff-Atome dieser Verbindung sich an die negativen Ladungen des Haar-Keratins anlagern kann; geschädigte Haare enthalten wegen ihres höheren Cysteinsäure-Gehalts mehr negativ geladene Säure-Gruppen und können daher mehr quartäre Ammonium-Verbindungen aufnehmen. Diese, wegen ihres kationaktiven Charakters auch als "kationaktive Pflegestoffe" bezeichnet, wirken glättend auf das Haar, verbessern die Kämmbarkeit, vermindern die elektrostatische Aufladung, verbessern Griff und Glanz. Die polymeren quartären Ammonium-Verbindungen haften so gut am Haar, daß ihre Wirkung noch nach mehreren Wäschen nachgewiesen werden kann. Organische Säuren wie Citronensäure, Weinsäure oder Milchsäure werden häufig zur Einstellung eines sauren Milieus eingesetzt. Die wasserlöslichen EiweißHydrolysate ziehen wegen ihrer engen chemischen Verwandtschaft gut auf das Haar-Keratin auf.

Die größte Gruppe spezielfer Wirkstoffe in Haarpflegemitteln bilden diverse Pflanzenextrakte und Pflanzenöle.
Üblicherweise werden diese Extrakte durch Extraktlon der gesamten Pflanze hergestellt. Es kann aber in einzelnen Fällen auch bevorzugt sein, die Extrakte ausschließlich aus Blüten und/oder Blättern der Pflanze herzustellen.

Hinsichtlich der erfindungsgemäß bevorzugten Pflanzenextrakte wird insbesondere auf die Extrakte hingewiesen, die in der auf Seite 44 der 3. Auflage des Leltfadens zur Inhaltsstoffdeklaration kosmetischer Mittel, herausgegeben vom Industrisverband Kärperpflege- und Waschmittel e.V. (IKW), Frankfurt, beginnenden Tabelle aufgeführt sind.

Erfingdungsgemäß sind vor allem die Extrakte aus Grünem Tee, Eichenrinde, Brennnessel, Hamamelis, Hopfen, Henna, Kamille, Klettenwurzel, Schachtelhalm, Weißdorn. Lindenblüten, Mandel, Aloe Vera, Fichtennadel, Rosskastanle, Sendelholz, Wacholder, Kokosnuss, Mango, Aprikose, Limone, Welzen, Kiwi, Melone, Orange, Grapefruit, Salbei, Rosmarin, Birke, Malve, Wiesenschaumkraut, Quendel, Schafgarbe, Thymian, Melisse, Hauhechel, Huflattich, Eibisch, Meristem, Ginseng und Ingwerwurzel bevorzugt.

Die Pflanzenextrakte können erfindungsgemäß sowohl in reiner als auch in verdünnter Form eingesetzt werden. Sofern sie in verdünnter Form eingesetzt werden, enthalten sie üblicherweise ca. 2 - 60 Gew.-% Aklivsubstanz und als Lösungsmittel das bei ihrer Gewinnung eingesetzte Extraktionsmittel oder Extraktionsmittelgemisch.

Weiterhin kann es bevorzugt sein, in den erfindungsgemäßen Mitteln Mischungen aus mehreren, insbesondere aus zwei, verschiedenen Pflanzenextrakten einzusetzen.

Zur Vermeidung einer zu schnellen Rückfettung enthalten einige Haarwässer Substanzen wie gewisse Teer-Inhaltsstoffe, Cysteinsäure-Derivate oder Glycyrrhizin; die beabsichtigte Verminderung der Talgdrüsenproduktion ist ebenfalls noch nicht eindeutig bewiesen. Dagegen ist die Wirksamkeit von Antischuppen-Wirkstoffen einwandfrei belegt. Sie werden daher in entsprechenden Haarwässern u.a. Pflegemitteln eingesetzt.

Bei den wässrigen Zubereitungen zur Behandlung von Haut handelt es sich insbesondere um Zubereitungen zur Pflege menschlicher Haut. Diese Pflege beginnt mit der Reinigung für die in erster Linie Seifen benutzt werden. Hier unterscheidet man feste, meist stückige und flüssige Seife. Dementsprechend liegen die kosmetischen Mittel in einer bevorzugten Ausführungsform als Formkörper vor, die oberflächenaktive Inhaltsstoffe enthalten. Wichtigste Inhaltsstoffe derartiger Formkörper sind in einer bevorzugten Ausführungsform die Alkalisalze der Fettsäuren natürlicher Öle und Fette, vorzugsweise mit Ketten von 12-18 C-Atomen. Da Laurinsäure-Seifen besonders gut schäumen, sind die Laurinsäure-reichen Kokos- und Palmkern-Öle bevorzugte Rohstoffe für die Feinseifen-Herstellung. Die Na-Salze der Fettsäure-Gemische sind fest, die K-Salze weichpastös. Zur Verseifung wird die verdünnte Natron- oder Kali-Lauge den Fett-Rohstoffen im stöchiometrichem Verhältnis so zugesetzt, daß in der fertigen Seife ein Laugenüberschuß von max. 0,05% vorhanden ist. Vielfach werden die Seifen heute nicht mehr direkt aus den Fetten, sondern aus den durch Fettspaltung gewonnenen Fettsäuren hergestellt. Übliche Seifen-Zusätze sind Fettsäuren, Fettalkohole, Lanolin, Lecithin, pflanzliche Öle, Partialglyceride u.a. fettähnliche Substanzen zur Rückfettung der gereinigten Haut, Antioxidantien wie Ascorbyl-Palmitat oder Tocopherol zur Verhinderung der Autoxidation der Seife (Ranzigkeit), Komplexierungsmittel wie Nitrilotriacetat zur Bindung von Schwermetall-Spuren, die den autoxidativen Verderb katalysieren könnten, Parfüm-Öle zur Erzielung der gewünschten Duftnoten, Farbstoffe zur Einfärbung der Seifenstücke und ggf. spezielle Zusätze.

Flüssige Seifen basieren sowohl auf K-Salzen natürlicher Fettsäuren als auch auf synthetischen Aniontensiden. Sie enthalten in wässriger Lösung weniger waschaktive Substanzen als feste Seifen, haben die üblichen Zusätze, ggf. mit viskositätsregulierenden Bestandteilen sowie Perlglanz-Additiven. Wegen ihrer bequemen und hygienischen Anwendung aus Spendern werden sie vorzugsweise in öffentlichen Waschräumen und dergleichen verwendet. Wasch-Lotionen für besonders empfindliche Haut basieren auf mild wirkenden synthetischen Tensiden mit Zusätzen hautpflegender Substanzen, pH-neutral oder schwach sauer (pH 5,5) eingestellt.

Zur Reinigung und Pflege vornehmlich der Gesichtshaut gibt es eine Reihe weitere Präparate, wie Gesichtswässer, Reinigungs-Lotionen, -Milche, -Cremes, -Pasten; Gesichtspackungen dienen z.T. der Reinigung, überwiegend jedoch der Erfrischung und Pflege der Gesichtshaut. Gesichtswässer sind meist wässrige-alkoholische Lösungen mit geringen Tensid-Anteilen sowie weiteren hautpflegenden Substanzen. Reinigungs-Lotionen, -Milche, -Cremes und -Pasten basieren meist auf O/W-Emulsionen mit relativ geringen Gehalten an Fettkomponenten mit reinigenden und pflegenden Zusätzen. Sogenannte Scruffing- und Peeling-Präparate enthalten mild keratolytisch wirkende Substanzen zur Entfernung der obersten abgestorbenen Haut-Horn-Schichten, z.T. mit Zusätzen abrasiv wirkender Pulver.

In Mitteln zur reinigenden Behandlung unreiner Haut sind außerdem antibakterielle und entzündungshemmende Substanzen enthalten, da die Talgansammlungen in Komedonen (Mitessern) Nährböden für bakterielle Infektionen darstellen und zu Entzündungen neigen. Die angebotene breite Palette-verschiedener Hautreinigungs-Produkte variiert in Zusammensetzung und Gehalt an diversen Wirkstoffen, abgestimmt auf die verschiedenen Hauttypen und auf spezielle Behandlungsziele.

Die für die Hautreinigung im Wannen- oder Duschbad angebotenen Badezusätze haben breite Anwendung gefunden. Badesalze und Badetabletten sollen das Badewasser enthärten, färben und parfümieren und enthalten in der Regel keine waschaktiven Substanzen. Durch die Enthärtung des Badewassers fördern sie die Reinigungskraft von Seifen, sollen jedoch in erster Linie erfrischend wirken und das Badeerlebnis verstärken. Größere Bedeutung haben die Schaumbäder. Bei einem höheren Gehalt an rückfettenden und hautpflegenden Substanzen spricht man auch von Creme-Bädern.

Die erfindungsgemäßen Kosmetika (kosmetischen Mittel) können in unterschiedlichen Zubereitungsformen vorliegen.

Die wichtigsten sind Haut-Cremes, -Lotionen, -Öle und -Gele. Basis der Cremes und Lotionen sind Emulsionen in O/W- (Öl in Wasser) od. W/O- (Wasser in Öl) Form. Die Hauptbestandteile der Öl- bzw. Fett- oder Lipid-Phase sind Fettalkohole, Fettsäuren, Fettsäureester, Wachse, Vaseline, Paraffine sowie weitere Fett- und Ölkomponenten hauptsächlich natürlichen Ursprungs. In der wässrigen Phase sind neben Wasser hauptsächlich feuchtigkeitsregulierende und feuchtigkeitsbewahrende Substanzen als wesentliche Hautpflege-Wirkstoffe enthalten, ferner konsistenz- bzw. viskositätsregulierende Mittel. Weitere Zusätze wie Konservierungsmittel, Antioxidantien, Komplexbildner, Parfüm-Öle, Färbemittel sowie spezielle Wirkstoffe werden je nach ihrer Löslichkeit und ihren Stabilitätseigenschaften einer der beiden vorgenannten Phasen beigegeben. Wesentlich für den Emulsionstyp und seine Eigenschaften ist die Auswahl des Emulgator-Systems. Seine Auswahl kann nach dem HLB-System erfolgen.

Weiterhin können die Hautpflegemittel weitere spezielle Wirkstoffe wie beispielsweise Milcheiweißprodukte, Eigelb Lecithine, Lipoide, Phosphatide, Getreidekeimöle, Vitamine - insbesondere Vitamin F und das früher als Hautvitamin (Vitamin H) bezeichnete Biotin sowie hormonfreie Placenta-Extrakte, enthalten.

Hautöle gehören zu den ältesten Produktformen der Hautpflege und werden noch heute verwendet. Basis sind nichttrocknende Pflanzenöle wie Mandelöl oder Olivenöl, mit Zusätzen natürlicher Vitaminöle wie Weizenkeimöl oder Avocadoöl sowie öligen Pflanzenextrakten aus z.B. Johanniskraut, Kamille u.ä..

Hautgele sind halbfeste transparente Produkte, die durch entsprechende Gelbildner stabilisiert werden. Man unterscheidet Oleogele (wasserfrei), Hydrogele (ölfrei) und Öl/Wasser-Gele. Die Typenauswahl richtet sich nach dem gewünschten Anwendungs-Zweck. Die Öl/Wasser-Gele enthalten hohe Emulgator-Anteile und weisen gegenüber Emulsionen gewisse Vorteile auf sowohl unter ästhetischen als auch unter Anwendungsgesichtspunkten.

Weitere erfindungsgemäß bevorzugte kosmetische Mittel sind Mittel zur Beeinflussung des Körpergeruchs. Insbesondere sind hier deodorierende Mittel gemeint. Derartige Deodorantien können Gerüche überdecken, entfernen oder zerstören. Unangenehme Körpergerüche entstehen bei bakterieller Zersetzung des Schweißes, insbesondere in den feuchtwarmen Achselhöhlen, wo Mikroorganismen gute Lebensbedingungen finden. Dementsprechend sind die wichtigsten Inhaltsstoffe von Deodorantien keimhemmende Substanzen. Insbesondere sind solche keimhemmenden Substanzen bevorzugt, die eine weitgehende selektive Wirksamkeit gegenüber den für den Körpergeruch verantwortlichen Bakterien besitzen. Bevorzugte Wirkstoffe haben dabei jedoch lediglich eine bakteriostatische Wirkung und töten die Bakterienflora keinesfalls ganz ab. Zu dem keimhemmenden Mitteln können generell alle geeigneten Konservierungsmittel mit spezifischer Wirkung gegen grampositiver Bakterien gerichtet werden. Beispielsweise sind dies Irgasan DP 300 (Trichlosan, 2,4,4'-Trichlor-2'-Hydroxydiphenylether), Chlorhexidin (1,1'-Hexamethylenbis(5-(4'-chlor-phenyl)-biguanid) sowie 3,4,4'-Trichlorcarbanilid. Auch quartäre Ammonium-Verbindungen sind prinzipiell ebenfalls geeignet. Aufgrund ihrer hohen antimikrobierenden Wirksamkeit werden all diese Stoffe bevorzugt nur in geringen Konzentrationen von etwa 0,1 bis 0,3 Gew.-% eingesetzt. Weiterhin haben auch zahlreiche Riechstoffe antimikrobielle Eigenschaften. Dementsprechend werden derartige Riechstoffe mit antimikrobiellen Eigenschaften in Deodorantien bevorzugt eingesetzt. Insbesondere sind hier Farnesol und Phenoxyethanol zu nennen. Daher ist es insbesondere bevorzugt, wenn die erfindungsgemäßen Deodorantien solche selbst bakteriostatisch wirksamen Riechstoffe enthalten. Dabei können die Riechstoffe vorzugsweise wieder in Form von Kieselsäureestern enthalten sein. Es ist jedoch auch möglich, daß gerade diese antibakteriell wirksamen Riechstoffe nicht in Form von Kieselsäureestern eingesetzt werden und dann in Mischungen mit anderen Riechstoffen, die als Kieselsäureester vorliegen, eingesetzt sind. Eine weitere Gruppe wesentlicher Inhaltsstoffe von Deodorantien sind Enzyminhibitoren, die die Zersetzung des Schweißes durch Enzyme hemmen, wie beispielsweise Citronensäuretriethylester oder Zinkglycinat. Wesentliche Inhaltsstoffe von Deodorantien sind weiterhin auch Antioxidantien, die eine Oxidation der Schweißbestandteile verhindern sollen.

Ein weiterer Gegenstand der vorliegenden Erfindung ist die Verwendung der erfindungsgemäßen Pro-Fragrances zur Verlängerung der Duftwirkung von Duftstoffen.

Aufgrund der hervorragenden Eignung der erfindungsgemäßen Verbindungen zum Einsatz in Wasch- und Reinigungsmitteln ist die Verwendung der erfindungsgemäßen Pro-Fragrances, in flüssigen oder festen Wasch- und Reinigungsmitteln, insbesondere bevorzugt als Duftstoff, ein weiterer Gegenstand der vorliegenden Erfindung.

Ebenso eignen sich die erfindungsgemäßen Pro-Fragrances hervorragend zum Einsatz in Kosmetika (kosmetischen Mitteln), daher ist ein weiterer Gegenstand der vorliegenden Erfindung die Verwendung der erfindungsgemäßen Pro-Fragrances in Kosmetika (kosmetischen Mitteln) zur Haut- und Haarbehandlung insbesondere bevorzugt als Duftstoff.

Ebenfalls ist ein Gegenstand der vorliegenden Erfindung die Verwendung der erfindungsgemäßen Pro-Fragrances zusammen mit anderen gängigen Duftstoffen, die vorzugsweise auf herkömmlicher Weise in Mittel, wie Wasch- und Reinigungsmittel sowie Weichspüler und Kosmetika, eingearbeitet werden.

Ein weiterer Gegenstand der vorliegenden Erfindung ist das Verfahren zur Verlängerung des Duftempfindens von Wasch- oder Reinigungsmitteln, Weichspülern oder Kosmetika oder mit diesen behandelten festen Oberflächen, dadurch gekennzeichnet, dass man die erfindungsgemäßen Pro-Fragrances oder Mischungen daraus zu den Wasch- oder Reinigungsmitteln, Weichspülern oder Kosmetika zugibt.
Die erfindungsgemäßen Pro-Fragrances setzen dabei vorzugsweise die darin derivatisierten Duftstoffe durch Hydrolyse nach und nach wieder frei.

Die erfindungsgemäßen Verbindungen und Mittel zeigen unter Umgebungsbedingungen eine gute hydrolytische Spaltbarkeit. Sie weisen zudem eine hohe Lagerstabilität in alkalischer Umgebung auf, wie sie beispielsweise in Waschmitteln und Geschirrreinigungsmitteln anzutreffen ist.

Die Erfindung betrifft zudem ein Verfahren zur Verlängerung des Duftempfindens von Wasch- oder Reinigungsmitteln, Weichspülern oder Kosmetika oder mit diesen Mitteln behandelten festen Oberflächen, bei dem man den Wasch- oder Reinigungsmitteln, Weichspülern oder Kosmetika erfindungsgemäße Verbindungen oder Mischungen einverleibt. Vorzugsweise werden die Duftstoffe dabei durch Hydrolyse wieder freigesetzt.

Die Erfindung wird durch die nachstehenden Beispiele näher erläutert.

### Synthese von 1-Aza-3,7-dioxa-bicyclo[3.3.0]octanen

### AA1: Allgemeine Arbeitsvorschrift zur Synthese von 1-Aza-3,7-dioxa-bicyclo[3.3.0]octanen, Verhältnis Aminoalkohol/Aldehyd 1:2

Der Aminoalkohol wird unter Stickstoffatmosphäre mit dem Aldehyd im Verhältnis 1:2 in Toluol vorgelegt. Das Reaktionsgemisch wird auf T = 120 °C erhitzt, wobei der Aminoalkohol langsam in Lösung geht. Rückflussen am Wasserabscheider für 7h. Einrotieren und Trocknen der klaren leicht gelblichen Lösung im Hochvakuum.

### AA2: Allgemeine Arbeitsvorschrift zur Synthese von 1-Aza-3,7-dioxa-bicyclo[3.3.0]octanen, Verhältnis Aminoalkohol/Aldehyd 1:2 in situ

Der Aminoalkohol wird unter Stickstoffatmosphäre mit dem Aldehyd im Verhältnis 1:2 vorgelegt. Das Reaktionsgemisch wird auf T = 100 °C-140°C erhitzt, wobei die Reaktanden langsam in Lösung gehen oder schmelzen. Das Reaktionsgemisch wird so lange erhitzt, bis kein Reaktionswasser mehr abdestilliert werden kann. Trocknen der klaren leicht gelblichen Lösung im Hochvakuum.

### Beispiel 1: Synthese von 1-Aza-3,7-dioxa-2,8-dioctyl-5-alkyl-bicyclo[3.3.0]octan

**a) mit R⁶ = H nach AA1: 1-Aza-3,7-dioxa-2,8-dioctyl-bicyclo[3.3.0]octan**
   2-Amino-1,3-propandiol m=1,37g, M=91,1g/mol, (15mmol),
   Octanal m=3,85g, M=128,21g/mol, (30mmol)
   Ausbeute=84%, m=3,95g gelbliche, klare Flüssigkeit, Reinheit: GC 90%ig
   **mit R⁶ = H nach AA2: 1-Aza-3,7-dioxa-2,8-dioctyl-bicyclo[3.3.0]octan**
   2-Amino-1,3-propandiol m=1,37g, M=91,1g/mol, (15mmol),
   Octanal m=3,85g, M=128,21g/mol, (30mmol)
   Ausbeute=88%, m=4,12 gelbliche, klare Flüssigkeit, Reinheit: GC 92%ig
   ¹H NMR (CDCl₃) charakteristische Signale des Bicyclus:

   δ 4,30 (dd; 2H), 4,08 (m, 2H), 3,80 (m, 1 H), 3,62 (m, 2H).
**b) mit R⁶ = Me nach AA1: 1-Aza-3,7-dioxa-2,8-dioctyl-5-methyl-bicyclo[3.3.0]octan**
   2-Amino-2-methyl-1,3-propandiol m=1,89g, M=105,13g/mol, (18mmol),
   Octanal m=4,61g, M=128,21g/mol, (36mmol)
   Ausbeute=98%, m=5,73g gelbliche, klare Flüssigkeit, Reinheit: GC 94%ig
   ¹H NMR (CDCl₃) charakteristische Signale des Bicyclus:
   δ 4,34 (dd, 2H), 3,71 (d, 2H), 3,63 (d, 2H).
**c) mit R⁶ = Hydroxymethyl** nach AA1: **1-Aza-3,7-dioxa-2,8-dioctyl-5-hydroxymethyl-bicyclo[3.3.0]octan**
   2-Amino-2-hydroxymethyl-1,3-propandiol m=1,82g, M=121,13g/mol, (15mmol),
   Octanal m=3,85g, M=128,21g/mol, (30mmol)
   Ausbeute=94%, m=4,81g klare hochviskose Flüssigkeit, Reinheit: GC 95,8%ig
   ¹H NMR (CDCl₃) charakteristische Signale des Bicyclus:
   δ 4,41 (dd, 2H), 3, 88 (d, 2H), 3,61 (d, 2H).
**d) mit** R⁶ **= Ethyl nach AA1: 1-Aza-3,7-dioxa-2,8-dioctyl-5-ethyl-bicyclo[3.3.0]octan**
   2-Amino-2-ethyl-1,3-propandiol m=3,57g, M=119,16g/mol, (30mmol),
   Octanal m=7,70g, M=128,21g/mol, (60mmol)
   Ausbeute=96%, m=9,74g klare hochviskose Flüssigkeit, Reinheit: GC 94,7%ig
   ¹H NMR (CDCl₃) charakteristische Signale des Bicyclus:
   δ 4,34 (dd, 2H), 3,80 (d, 2H), 3,57 (d, 2H).

### Beispiel 2: Synthese von 1-Aza-3,7-dioxa-2,8-di (2,6-dimethyl-5-heptenyl)-5-alkyl-bicyclo[3.3.0]octan

**a) mit R⁶ = H nach AA1: 1-Aza-3,7-dioxa-2,8-di (2,6-dimethyl-5-heptenyl)-bicyclo[3.3.0]octan**
   2-Amino-1,3-propandiol m=1,37g, M=91,1g/mol, (15mmol)
   2,6-Dimethylheptyl-5-enal m=5,34g, M=178,12g/mol, (30mmol)
   Ausbeute=94%, m=7,1g gelbliche, klare Flüssigkeit. Reinheit: GC 86%ig
   ¹H NMR (CDCl₃) charakteristische Signale des Bicyclus:
   δ 4,24 (dd, 2H), 4,04 (m, 2H), 3,68 (m, 1H), 3,58 (m, 2H).
**b) mit R⁶ = Me nach AA1: 1-Aza-3,7-dioxa-2,8-di(2,6-dimethyl-5-heptenyl)-5-methyl-bicyclo[3.3.0]octan**
   2-Amino-2-methyl-1,3-propandiol m=1,78g, M=105,13g/mol_{,} (17mmol),
   2,6-Dimethylheptyl-5-enal m=6,05g, M=178,12g/mol, (34mmol)
   Ausbeute=85%, m=5,41g gelbliche, klare Flüssigkeit , Reinheit: GC 87%ig
   ¹H NMR (CDCl₃) charakteristische Signale des Bicyclus:
   δ 4,12 (m, 2H), 3,64 (m, 2H), 3,46 (m, 2H).
**c) mit R³ = Hydroxymethyl nach AA1: 1-Aza-3,7-dioxa-2,8-di(2,6-dimethyl-5-heptenyl)-5-hydroxymethyl-bicyclo[3.3.0]octan**
   2-Amino-2-hydroxymethyl-1,3-propandiol m=1,82g, M=121,13g/mol, (15mmol),
   2,6-Dimethylheptyl-5-enal m=5,34g, M=178,12g/mol, (30mmol)
   Ausbeute=97%, m=5,35g klare hochviskose Flüssigkeit , Reinheit: GC 99,1%ig
   ¹H NMR (CDCl₃) charakteristische Signale des Bicyclus:
   δ 4,29 (dd, 2H), 4,18 (m, 2H).
**d) mit R⁶ = Ethyl nach AA1: 1-Aza-3,7-dioxa-2,8-di(2,6-dimethyl-5-heptenyl)-5-ethyl-bicyclo[3.3.0]octan**
   2-Amino-2-ethyl-1,3-propandiol m=5,21g, M=119,16g/mol, (43,7mmol),
   2,6-Dimethylheptyl-5-enal m=12,3g, M=128,21g/mol_{,} (87,4mmol)
   Ausbeute=79%, m=12,51g g klare hochviskose Flüssigkeit, Reinheit: GC 92,9%ig
   ¹H NMR (CDCl₃) charakteristische Signale des Bicyclus:
   δ 4,16 (m, 2H), 3,69 (m, 2H), 3,51 (m, 2H).

### Beispiel 3: Synthese von 1-Aza-3,7-dioxa-2,8-di (3-(4-tert-butylphenyl)butyl)-5-alkyl-bicyclo[3.3.0]octan

**a) mit R⁶ = H nach AAV1: 1-Aza-3,7-dioxa-2,8-di (3-(4-tert-butylphenyl)butyl)-bicyclo[3.3.0]octan**
   2-Amino-1,3-propandiol m=1,37g, M=91,1g/mol, (15mmol)
   3-(4-tert-butylphenyl)butanal m=6,12g, M=204,3/mol, (30mmol)
   Ausbeute=100%, m=6,9g gelbliche, klare Flüssigkeit , Reinheit: GC 90%ig
   ¹H NMR (CDCl₃) charakteristische Signale des Bicyclus:
   δ 4,34 (dd, 2H), 4,12 (m, 2H), 3,82 (m, 1H), 3,66 (m, 2H).
**b) mit R⁶ = Me nach AA1: 1-Aza-3,7-dioxa-2,8-di (3-(4-tert-butylphenyl)butyl)-5-methyl-bicyclo[3.3.0]octan**
   2-Amino-2-methyl-1,3-propandiol m=1,37g, M=105,13g/mol, (13mmol),
   3-(4-tert-butylphenyl)butanal m=5,31g, M=204,3/mol, (26mmol)
   Ausbeute=97%, m=6,04g gelbliche, klare Flüssigkeit, Reinheit: GC 93%ig
   ¹H NMR (CDCl₃) charakteristische Signale des Bicyclus:
   δ 4,24 (dd, 2H), 4,16 (m, 2H).
**c) mit R⁶ = Hydroxymethyl nach AA1: 1-Aza-3,7-dioxa-2,8-di (3-(4-tert-butylphenyl)butyl)-5-hydoxymethyl-bicyclo[3.3.0]octan**
   2-Amino-2-hydroxymethyl-1,3-propandiol m=1,82g, M=121,13g/mol, (15mmol),
   3-(4-tert-butylphenyl)butanal m=6,12g, M=204,3/mol, (30mmol)
   Ausbeute=97%, m=7,19g klare hochviskose Flüssigkeit, Reinheit: GC 93%ig
   ¹H NMR (CDCl₃) charakteristische Signale des Bicyclus:
   δ 4,28 (dd, 2H), 4,19 (m, 2H).
**d) mit R⁶ = Ethyl nach AA1: 1-Aza-3,7-dioxa-2,8-di (3-(4-tert-butylphenyl)butyl)-5-ethyl-bicyclo[3.3.0]octan**
   2-Amino-2-ethyl-1,3-propandiol m=1,78g, M=119,16g/mol, (15mmol),
   3-(4-tert-butylphenyl)butanal m=6,12g, M=204,3/mol, (30mmol)
   Ausbeute=89%, m=6,60g klare hochviskose Flüssigkeit, Reinheit: GC 88%ig
   ¹H NMR (CDCl₃) charakteristische Signale des Bicyclus:
   δ 4,26 (m, 1 H), 4,18 (dd, 1 H), 3,85 (m, 2H), 3,63 (m, 2H).

### Beispiel 4: Synthese von 1-Aza-3,7-dioxa-2-phenylacetyl-8-octyl -bicyclo[3.3.0]octan

### 1. Stufe nach AAV1:

2-Amino-1,3-propandiol m=1,37g, M=91,1g/mol, (15mmol)
1-Phenylethanon m=5,79g, M=193,24g/mol, (30mmol)
t=24h, Einrotieren der anfangs bläulichen Lösung und Trocknen im Hochvakuum, danach ist die Lösung leicht gelblich und klar.
Einfach substituiertes Oxazolidin.
Ausbeute=55%, m=2,2g gelbliche, klare Flüssigkeit, Reinheit: GC 98,5%ig.
¹H NMR (CDCl₃): □ 7,59 (dd, 2H), 7,34 (m, 3H), 4,11 (dd, 0,5 H), 3,76 (m, 2,5H), 3,42 (dd, 0,5 H), 3,30 (m, 1 H), 3,06 (m, 0,5H), 1,69 (s, 1,5 H), 1,66 (s, 1,5H).

### 2. Stufe nach AAV1:

[2-Methyl-2-phenyl-1,3-oxazolidin-4-yl]methanol m = 1,19g, M = 193,24 g/mol (5,7mmol)
Octanal m=0,73g, M=128,21g/mol, (5,7mmolmmol)
Ausbeute=99,9%, m=1,83g gelbliche, klare Flüssigkeit, Reinheit: GC 80%ig
¹H NMR (CDCl₃) charakteristische Signale des Bicyclus:
δ 4,90 (dd, 2H), 4,38 (dd, 1 H), 4,10 (m, 2H), 4,05 (dd, 1 H).

### Riechtest

Für den im Folgenden beschriebenen Riechtest wurden 0,2 mmol des Riechstoffderivats in Dichlormethan oder Ethanol 1 ml Lösungsmittel gelöst. In die Lösung wurde ein Riechstreifen 2 cm tief eingetaucht und dann trocknen gelassen.

Als Referenz beim Dufteindruck diente stets der underivatisierte Riechstoff. Hierfür wurde der Riechstoff ebenfalls in einer Menge von 0,2 mmol in 1 ml Ethanol gelöst. In die Lösung wurde ein Riechstreifen 2 cm tief eingetaucht und dann trocknen gelassen.

Die Riechstreifen wurden einmal im trockenen Zustand und täglich feucht/besprüht abgerochen. Zum Besprühen dienten Wasser (pH=7) und die entsprechenden Pufferlösungen mit pH 6-1. Die Duftintensität wurde von 3 trainierten Probanden auf einer Skala von 0 bis 4 bewertet, wobei 4 die höchste Note ist und 0 für keine Duftwahrnehmung steht.

### Definition der Skalierung:

- 4: stark
- 3: intensiv
- 2: angenehm
- 1: wahrnehmbar
- 0: nicht mehr wahrnehmbar

| 1- 1-Aza-3,7-dioxa-2,8-dioctyl-5-alkyl-bicyclo[3.3.0]octan | trocken | pH = 7 H₂O | pH=4 | pH = 2 | pH = 1 | Octanal mit Wasser | Octanal trocken |
|---|---|---|---|---|---|---|---|
| Tag 0 | 2 | 1-2 | 2 | 2 | 4 | 2 | 2 |
| Tag 1 | 2 | 1-2 | 2 | 2 | 3 | 1 | 1 |
| Tag 2 | 2 | 1-2 | 2 | 2 | 3 | 1 | 0-1 |
| Tag 3 | 1 | 1-2 | 1-2 | 1-2 | 2-3 | 1 | 0-1 |
| Tag 7 | 1 | 1-2 | 1-2 | 1-2 | 2 | 1 | 0 |

| **Synthese von** 1-**Aza-3,7-dioxa-2,8-di (2,6-dimethyl-5-heptenyl)-5-alkyl-bicyclo[3.3.0]octan** | trocken | pH = 7 H₂O | pH = 4 | pH = 2 | pH = 1 | 2,6-Dimethylheptyl-5-enal mit Wasser | 2,6-Dimethylheptyl-5-enal trocken |
|---|---|---|---|---|---|---|---|
| Tag 0 | 1 | 2-3 | 2-3 | 2-3 | 2-3 | 2 | 1-2 |
| Tag 1 | 1 | 2 | 2 | 2 | 2-3 | 0 | 0 |
| Tag 2 | 0-1 | 2 | 2 | 2 | 2-3 | 0 | 0 |
| Tag 3 | 0-1 | 1 | 1-2 | 2 | 2-3 | 0 | 0 |
| Tag 7 | 0-1 | 1 | 1-2 | 2 | 2-3 | 0 | 0 |

| **1-Aza-3,7-dioxa-2,8-di** (**3-(4-tert-butylphenyl) butyl)-5-alkyl-bicyclo[3.3.0] octan** | trocken | pH = 7 H₂O | pH =4 | pH =2 | pH =1 | 3-(4-tert-butylphenyl)butanal mit Wasser | 3-(4-tert-butylphenyl)butanal trocken |
|---|---|---|---|---|---|---|---|
| Tag 0 | 3 | 3 | 3 | 3 | 3 | 2-3 | 2 |
| Tag 1 | 2 | 2 | 2 | 2 | 2 | 2 | 1 |
| Tag 2 | 1 | 1-2 | 1 -2 | 1 | 2 | 1 | 0 |
| Tag 3 | 1 | 1 | 1 | 1 | 1 -2 | 0-1 | 0 |
| Tag 7 | 0-1 | 1 | 1 | 1 | 1 -2 | 0-1 | 0 |

## Patentansprüche

1. 1-Aza-3,7-dioxabicyclo[3.3.0]octan-Verbindung der allgemeinen Formel (I) 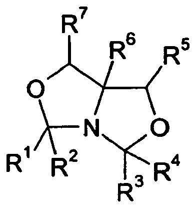 mit der Bedeutung
R¹, R², R³, R⁴ unabhängig voneinander Reste, die in einer Verbindung der allgemeinen Formel R¹-C(=O)-R² bzw. R³-C(=O)-R⁴ einen Duftaldehyd oder ein Duftketon ergeben, wobei R¹ und R² bzw. R³ und R⁴ nicht gleichzeitig Wasserstoff sein können,
R⁶ H, Alkyl, das durch zwei Hydroxylgruppen und/oder eine Aminogruppe substituiert sein kann und/oder in dem bis zu 8 nicht benachtbarte -CH₂-Gruppen durch -O-ersetzt sein können,
R⁵, R⁷ unabhängig voneinander H oder C₁₋₆-Alkyl,
oder Gemische dieser Verbindungen,
wobei das genannte Keton ausgewählt ist aus der Gruppe, bestehend aus Buccoxim; Iso jasmon; Methyl beta naphthyl keton; Moschus indanon; Tonalid/Moschus plus; Alpha-Damascon, Beta-Damascon, Delta-Damascon, Iso-Damascon, Damascenon, Damarose, Methyl-dihydrojasmonat, Menthon, Carvon, Campher, Fenchon, Alpha-Ionen, Beta-Ionon, Dihydro-Beta-Ionon, Gamma-Methyl so genanntes Ionon, Fleuramon, Dihydrojasmon, Cis-Jasmon, Iso-E-Super, Methyl-cedrenyl-keton oder Methyl-cedrylon, Acetophenon, Methyl-acetophenon, Para-Methoxy-acetophenon, Methyl-beta-naphtyl-keton, Benzyl-aceton, Benzophenon, Para-Hydroxy-phenyl-butanon, Celery Keton oder Livescon, 6-Isopropyldecahydro-2-naphton, Dimethyl- octenon, Freskomenth, 4-(I-Ethoxyvinyl)-3,3,5,5,-tetramethyl-cyclohexanon, Methyl-heptenon, 2-(2-(4-Methyl-3-cyclohexen-1-yl)propyl)-cyclopentanon, 1-(p-Menthen-6(2)-yl)-1-propanon, 4-(4-Hydroxy-3-methoxyphenyl)-2-butanon, 2-Acetyl-3,3-dimethyl-norbornan, 6,7-Dihydro-1,1,2,3,3-pentamethyl-4(5H)-indanon, 4-Damascol, Dulcinyl or Cassion, Gelson, Hexalon, Isocyclemon E, Methyl-cyclocitron, Methyl-Lavendel-keton, Orivon, Para-tert-butyl-cyclohexanon, Verdon, Delphon, Muscon, Neobutenon, Plicaton, Velouton, 2,4,4,7-Tetramethyl-oct-6-en-3-on, Tetrameran, Hedion und Gemischen davon,
und wobei das genannte Aldehyd ausgewählt ist aus Melonal, Triplal, Ligustral, Adoxal; Anisaldehyd; Cymal; Ethylvanillin; Florhydral; Helional; Heliotropin; Hydroxycitronellal; Koavon; Laurinaldehyd; Lyral; Methyl-nonyl-acetaldehyd; p,t-Bucinal; Phenylacetaldehyd; Undecylenaldehyd; Vanillin;2,6,10-Trimethyl-9-undecenal, 3-Dodecen-I-al, alpha-n-Amylzimtaldehyd, 4-Methoxybenzaldehyd, Benzaldehyd, 3-(4-tert-Butylphenyl)-propanal, 2-Methyl-3-(para-methoxyphenylpropanal), 2-Methyl-4-(2,6,6-trimethyl-2(1)-cyclohexen-1-yl)butanal, 3-Phenyl-2-propenal, cis-/trans-3,7-Dimethyl-2,6-octadien-1-al, 3,7-Dimethyl-6-octen-I-al, [(3,7-Dimethyl-6-octenyl)oxy]acetaldehyd, 4-Isopropylbenzyaldehyd, 1,2,3,4,5,6,7,8-octahydro-8,8-Dimethyl-2-naphthaldehyd, 2,4-Dimethyl-3-cyclohexen-1-carboxyaldehyd, 2-Methyl-3-(isopropylphenyl)propanal,Decyl aldehyd, 2,6-Dimethyl-5-heptenal; 4-(tricyclo[5.2. 1.0 (2,6)]-decylidene-8)-butanal; Octahydro-4,7-methano-1H-indenecarboxaldehyd; 3-Ethoxy-4-hydroxybenzaldehyd, para-Ethyl-alpha, alpha-dimethylhydrozimtaldehyd, alpha-Methyl-3,4-(methylenedioxy)-hydrozimtaldehyd, 3,4-Methylenedioxybenzaldehyd, alpha-n-Hexylzimtaldehyd, m-Cymene-7-carboxaldehyd, alpha-Methylphenylacetaldehyd, 7-Hydroxy-3,7-dimethyl octanal, Undecenal, 2,4,6-Trimethyl-3-cyclohexene-1-carboxaldehyd, 4-(3)(4-Methyl-3-pentenyl)-3-cyclohexen-carboxaldehyd, 1-Dodecanal, 2,4-Dimethyl cyclohexene-3-carboxaldehyd, 4-(4-Hydroxy-4-methyl pentyl)-3-cylohexene-1-carboxaldehyd, 7-Methoxy-3,7-dimethyloctan-1-al, 2-Methyl undecanal, 2-Methyl decanal,1-nonanal, 1-Octanal, 2,6,10-Trimethyl-5,9-undecadienal, 2-Methyl-3-(4-tertbutyl)propanal, Dihydrozimtaldehyd, 1-methyl-4-(4-methyl-3-pentenyl)-3-cyclohexene-1-carboxaldehyd, 5 oder 6 Methoxyhexahydro-4,7-methanoindan-1 oder 2-carboxyaldehyd; 3,7-Dimethyloctan-1-al, 1-Undecanal,10-Undecen-1-al, 4-Hydroxy-3-methoxybenzaldehyd, 1-Methyl-3-(4-methylpentyl)-3-cyclohexenecarboxyaldehyd, 7-Hydroxy-3,7-dimethyl-octanal; trans-4-decenal, 2,6-Nonadienal, para-Tolylacetaldehyd; 4-Methylphenylacetaldehyd, 2-Methyl-4-(2,6,6-trimethyl-1-cyclohexen-1-yl)-2-butenal, ortho-Methoxyzimtaldehyd, 3,5,6-Trimethyl-3-cyclohexenecarboxaldehyd; 3,7-Dimethyl-2-methylene-6-octenal, Phenoxyacetaldehyd; 5,9-Dimethyl-4,8-decadienal, Peony aldehyd (6,10-dimethyl-3-oxa-5,9-undecadien-1-al), Hexahydro-4,7-methanoindan-1-carboxaldehyd, 2-Methyl octanal, alpha-Methyl-4-(l-methylethyl) benzeneacetaldehyd, 6,6-Dimethyl-2-norpinene-2-propionaldehyd, para Methyl phenoxy acetaldehyd; 2-methyl-3-phenyl-2-propen-1-al, 3,5,5-Trimethylhexanal, Hexahydro-8,8-dimethyl-2-naphthaldehyd, 3-Propyl-bicyclo [2.2.1]-hept-5-ene-2-carbaldehyd, 9-Decenal, 3-Methyl-5-phenyl-1-pentanal, Methylnonyl acetaldehyd, 1-p-Menthene-q-carboxaldehyd, Citral oder Gemische davon, Lilial, Citral, 1-Decanal, Florhydral, 2,4-Dimethyl-3-cyclohexen-1-carboxaldehyd.

2. Verbindung nach Anspruch 1, **dadurch gekennzeichnet, dass** maximal in einem der Strukturelemente -CR¹R² bzw. -CR³R⁴ Reste R¹ und R² bzw. R³ und R⁴ vorliegen, die in einer Verbindung der allgemeinen Formel R¹C(=O)-R² bzw. R³-C(=O)-R⁴ ein Duftketon ergeben.

3. Verbindung nach einem der Ansprüche 1 oder 2, **dadurch gekennzeichnet, dass** R², R⁴, R⁵, R⁶, R⁷ Wasserstoff bedeuten und R¹ und R³ jeweils einen C₄₋₂₄-Kohlenwasserstoffrest bedeuten.

4. Mischung aus Verbindungen der allgemeinen Formel (I) gemäß einem der Ansprüche 1 bis 3 und Verbindungen der allgemeinen Formel (II) 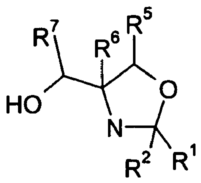 mit den in der allgemeinen Formel (I) angegebenen Bedeutungen für R¹, R², R⁵, R⁶, R⁷.

5. Verfahren zur Herstellung von Verbindungen nach einem der Ansprüche 1 bis 3 oder Mischungen nach Anspruch 4 durch Umsetzung von Verbindungen der allgemeinen Formel (III) 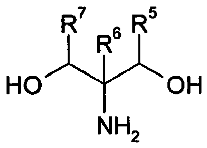 mit Verbindungen der allgemeinen Formel R¹-C(=O)-R² und R³-C(=O)-R⁴ unter Ringschluss.

6. Verwendung von Verbindungen nach einem der Ansprüche 1 bis 3 oder Mischungen nach Anspruch 4 als Pro-Fragrances.

7. Verwendung nach Anspruch 6, **dadurch gekennzeichnet, dass** die Verbindungen Duftstoffaldehyde als Duftstoffe freisetzen.

8. Verwendung nach Anspruch 6, **dadurch gekennzeichnet, dass** die Verbindungen Duftstoffketone als Duftstoffe freisetzen.

9. Verwendung nach einem der vorhergehenden Ansprüche 6 bis 8, **dadurch gekennzeichnet, dass** die Verbindungen zusammen mit anderen Duftstoffen eingesetzt werden.

10. Verwendung nach einem der vorhergehenden Ansprüche 6 bis 9 in Wasch- und Reinigungsmitteln, Weichspülern und Kosmetika.

11. Wasch- oder Reinigungsmittel, Weichspüler oder Kosmetika, enthaltend Verbindungen nach einem der Ansprüche 1 bis 3 oder Mischungen nach Anspruch 4.

12. Wasch- oder Reinigungsmittel, Weichspüler oder Kosmetika nach Anspruch 11, **dadurch gekennzeichnet, dass** die 1-Aza-3,7-dioxabicyclo[3.3.0]octan-Verbindung oder Mischungen daraus in Mengen von weniger als 5 Gew.%, vorzugsweise weniger als 2 Gew.-%, insbesondere weniger als 1 Gew.-%, jeweils bezogen auf die Gesamtmenge der Mittel, in dem Mittel enthalten ist.

13. Wasch- oder Reinigungsmittel, Weichspüler oder Kosmetika, nach einem der vorhergehenden Ansprüche 11 oder 12, **dadurch gekennzeichnet, dass** es sich um feste, flüssige oder gelförmige Formulierungen handelt.

14. Wasch- oder Reinigungsmittel nach Anspruch 13, **dadurch gekennzeichnet, dass** es sich bei festen Formulierungen um Pulver-, Granulat-, Tabletten- oder Tab-Formen und bei flüssigen Formulierungen um Lösungen, Emulsionen oder Dispersionen handelt.

15. Wasch- oder Reinigungsmittel nach einem der vorhergehenden Ansprüche 11 bis 14, **dadurch gekennzeichnet, dass** es sich um Reiniger aus der Gruppe der flüssigen oder gelförmigen Reiniger für harte Oberflächen, insbesondere den sogenannten Allzweckreinigern, Glasreinigern, Boden- oder Badezimmerreinigern, sowie spezielle Ausführungsformen derartiger Reiniger wozu saure oder alkalische Formen von Allzweckreinigern ebenso wie Glasreiniger mit sogenannter Anti-Rain-Wirkung gehören, handelt.

16. Kosmetisches Mittel nach einem der vorhergehenden Ansprüche 11 bis 13, **dadurch gekennzeichnet, dass** es sich um eine wässrige Zubereitung, die oberflächenaktive Wirkstoffe enthält und sich insbesondere zur Behandlung von Keratinfasern, insbesondere menschlichen Haaren, oder zur Behandlung von Haut eignet, handelt.

17. Kosmetisches Mittel nach einem der vorhergehenden Ansprüche 11 bis 13, **dadurch gekennzeichnet, dass** es sich um ein Mittel zur Beeinflussung des Körpergeruchs, insbesondere um ein deodorierendes Mittel handelt.

18. Verfahren zur Verlängerung des Duftempfindens von Wasch- oder Reinigungsmitteln, Weichspülern oder Kosmetika oder mit diesen behandelten festen Oberflächen, **dadurch gekennzeichnet, dass** man den Wasch- oder Reinigungsmitteln, Weichspülern oder Kosmetika Verbindungen nach einem der Ansprüche 1 bis 3 oder Mischungen nach Anspruch 4 einverleibt.

19. Verfahren nach Anspruch 18, wobei der Duftstoff durch Hydrolyse wieder freigesetzt wird.

## Claims

1. 1-Aza-3,7-dioxabicyclo[3.3.0]octane compound of the general Formula (I) 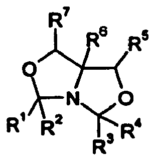 with the meaning
R¹, R², R³, R⁴ independently of one another are groups that afford a scent aldehyde or a scent ketone in a compound of the general Formula R¹-C(=O)-R² or R³-C(=O)-R⁴, wherein R¹ and R² or R³ and R⁴ may not simultaneously be hydrogen,
R⁶ denotes H, alkyl, which may be substituted by two hydroxyl groups and/or an amino group and/or in which up to 8 non-neighbouring -CH₂- groups may be replaced by -O-,
R⁵, R⁷ independently of one another denote H or C₁ to C₆ alkyl
or mixtures of these compounds,
wherein the cited ketone is selected from the group consisting of buccoxime; iso jasmone; methyl beta naphthyl ketone; musk indanone; Tonalid/Musk plus; alpha-damascone, beta-damascone, deltadamascone, iso-damascone, damascenone, damarose, methyl dihydrojasmonate, menthone, carvone, campher, fenchone, alphaionone, beta-ionone, dihydrobeta-ionone, gamma-methyl "ionone", fleuramone, dihydrojasmone, cis-jasmone, iso-E-Super, methyl cedrenyl ketone or methyl cedrylone, acetophenone, methyl acetophenone, para-methoxy acetophenone, methyl beta-naphthyl ketone, benzyl acetone, benzophenone, *para*-hydroxyphenyl butanone, celery ketone or livescone, 6-isopropyldecahydro-2-naphthone, dimethyloctenone, freskomenth, 4-(1-ethoxyvinyl)-3,3,5,5,-tetramethyl-cyclohexanone, methyl heptenone, 2-(2-(4-methyl-3-cyclohexen-1-yl)propyl)-cyclopentanone, 1-(*p*-menthen-6(2)-yl)-1-propanone, 4-(4-hydroxy-3-methoxyphenyl)-2-butanone, 2-acetyl-3,3-dimethyl-norbornane, 6,7-dihydro-1,1,2,3,3-pentamethyl-4(5*H*)-indanone, 4-damascol, dulcinyl or cassione, gelsone, hexalone, isocyclemone E, methylcyclocitrone, methyl lavendel ketone, orivone, para-tert-butylcyclohexanone, verdone, delphone, muscone, *neo*-butenone, plicatone, veloutone, 2,4,4,7-tetramethyl-oct-6-en-3-one, tetrameran, hedione and mixtures thereof and wherein the cited aldehyde is selected from melonal, triplal, ligustral, adoxal; anisaldehyde; cymal; ethyl vanillin; florhydral; helional; heliotropine; hydroxycitronellal; coavone; lauric aldehyde; lyral; methylnonyl acetaldehyde; *p*-t-bucinal; phenyl acetaldehyde; undecylene aldehyde; vanillin; 2,6,10-trimethyl-9-undecenal, 3-dodecen-1-al, alpha-*n*-amyl cinnamaldehyde, 4-methoxybenzaldehyde, benzaldehyde, 3-(4-*tert*-butylphenyl)-propanal, 2-methyl-3-(*para*-methoxyphenylpropanal), 2-methyl-4-(2,6,6-trimethyl-2(1)-cyclohexen-1-yl)butanal, 3-phenyl-2-propenal, *cis*/*trans*-3,7-dimethyl-2,6-octadien-1-al, 3,7-dimethyl-6-octen-1-al, [(3,7-dimethyl-6-octenyl)oxy]acetaldehyde, 4-isopropylbenzyaldehyd, 1,2,3,4,5,6,7,8-octahydro-8,8-dimethyl-2-naphthaldehyde, 2,4-dimethyl-3-cyclohexen-1-carboxyaldehyde, 2-methyl-3-(isopropylphenyl)propanal, decyl aldehyde, 2,6-dimethyl-5-heptenal; 4-(tricyclo[5.2.1.0(2,6)]-decylidene-8)-butanal; octahydro-4,7-methano-1*H*-indenecarboxaldehyde; 3-ethoxy-4-hydroxybenzaldehyde, *para*-ethyl-alpha,alpha-dimethylhydrocinnamaldehyde, alpha-methyl-3,4-(methylenedioxy)-hydrocinnamaldehyde, 3,4-methylenedioxybenzaldehyde, alpha-*n*-hexylcinnamaldehyde, *m-*cymene-7-carboxaldehyde, alpha-methylphenylacetaldehyde, 7-hydroxy-3,7-dimethyl octanal, undecenal, 2,4,6-trimethyl-3-cyclohexene-1-carboxaldehyde, 4-(3)(4-methyl-3-pentenyl)-3-cyclohexenecarboxaldehyde, 1-dodecanal, 2,4-dimethylcyclohexene-3-carboxaldehyde, 4-(4-hydroxy-4-methyl pentyl)-3-cylohexene-1-carboxaldehyde, 7-methoxy-3,7-dimethyloctan-1-al, 2-methyl undecanal, 2-methyl decanal, 1-nonanal, 1-octanal, 2,6,10-trimethyl-5,9-undecadienal, 2-methyl-3-(4-*tert*-butyl)propanal-dihydrocinnamaldehyde, 1-methyl-4-(4-methyl-3-pentenyl)-3-cyclohexene-1-carboxaldehyde, 5- or 6-methoxyhexahydro-4,7-methanoindane-1 or 2-carboxyaldehyde; 3,7-dimethyloctan-1-al, 1-undecanal, 10-undecen-1-al, 4-hydroxy-3-methoxybenzaldehyde, 1-methyl-3-(4-methylpentyl)-3-cyclohexene carboxyaldehyde, 7-hydroxy-3,7-dimethyl-octanal; trans-4-decenal, 2,6-nonadienal, paratolylacetaldehyde; 4-methylphenylacetaldehyde, 2-methyl-4-(2,6,6-trimethyl-1-cyclohexen-1-yl)-2-butenal, *ortho*-methoxycinnamaldehyde, 3,5,6-trimethyl-3-cyclohexene carboxaldehyde; 3,7-dimethyl-2-methylene-6-octenal, phenoxyacetaldehyde; 5,9-dimethyl-4,8-decadienal, peony aldehyde (6,10-dimethyl-3-oxa-5,9-undecadien-1-al), hexahydro-4,7-methanoindane-1-carboxaldehyde, 2-methyl octanal, alpha-methyl-4-(1-methylethyl)benzene acetaldehyde, 6,6-dimethyl-2-norpinene-2-propionaldehyde, *para*-methylphenoxy acetaldehyde; 2-methyl-3-phenyl-2-propen-1-al, 3,5,5-trimethylhexanal, hexahydro-8,8-dimethyl-2-naphthaldehyde, 3-propyl-bicyclo [2.2.1]-hept-5-ene-2-carbaldehyde, 9-decenal, 3-methyl-5-phenyl-1-pentanal, methyl-nonyl acetaldehyde, 1-*p*-menthene-*q*-carboxaldehyde, citral or mixtures thereof, lilial citral, 1-decanal, florhydral, 2,4-dimethyl-3-cyclohexene-1-carboxaldehyde.

2. The compound according to claim 1, **characterised in that** in one at the most of the structural elements -CR¹R² or -CR³R⁴ groups R¹ and R² or R³ and R⁴ are present, which afford a scent ketone in a compound of the general Formula R'-C(=O)-R² and/or R³-C(=O)-R⁴.

3. The compound according to one of the claims 1 or 2, **characterised in that** R², R⁴, R⁵, R⁶, R⁷ denote hydrogen and R¹ and R³ each denote a C₄₋₂₄ hydrocarbon group.

4. A mixture of the compounds of the general Formula (I) according to one of the claims 1 to 3 and compounds of general Formula (II) 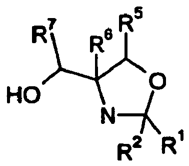 with the meanings given in general Formula (I) for R¹, R², R⁵, R⁶, R⁷.

5. A method for preparing compounds according to one of the claims 1 to 3 or mixtures according to claim 4 by reacting compounds of the general Formula (III) 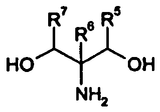 with compounds of general Formula R¹-C(=O)-R² and R³-C(=O)-R⁴ with ring closure.

6. Use of compounds according to any of the claims 1 to 3 or mixtures according to claim 4 as pro-fragrances.

7. The use according to claim 6, **characterised in that** the compounds release scent aldehydes as scent substances.

8. The use according to claim 6, **characterised in that** the compounds release scent ketones as scent substances.

9. The use according to one of the preceding claims 6 to 8, **characterised in that** the compounds are used together with other scent substances.

10. The use according to any one of the preceding claims 6 to 9 in washing and cleaning agents, fabric softeners and cosmetics.

11. Washing or cleaning agents, fabric softeners or cosmetics comprising compounds according to one of the claims 1 to 3 or mixtures according to claim 4.

12. The washing or cleaning agents, fabric softeners or cosmetics according to claim 11, **characterised in that** the 1-aza-3,7-dioxabicyclo[3.3.0]octane compound or mixtures thereof is comprised in the agent in amounts of less than 5 wt %, preferably less than 2 wt %, in particular less than 1 wt %, each based on the total amount of the agents.

13. The washing or cleaning agents, fabric softeners or cosmetics according to one of the preceding claims 11 or 12, **characterised in that** they are solid, liquid or gel formulations.

14. The washing or cleaning agents according to claim 13, **characterised in that** for solid formulations they are powder, granule, tablet or tab forms and for liquid formulations they are solutions, emulsions or dispersions.

15. The washing or cleaning agents according to one of the preceding claims 11 to 14, **characterised in that** they concern cleaners from the group of liquid or gel cleaners for hard surfaces, in particular the so-called all-purpose cleaners, glass cleaners, floor or bathroom cleaners, as well as special embodiments of such cleaners, which include acidic or alkaline forms of all-purpose cleaners as well as glass cleaners with a so-called anti-rain effect.

16. A cosmetic agent according to one of the preceding claims 11 to 13, **characterised in that** it concerns an aqueous preparation that comprises surface-active active substances and are suitable in particular for the treatment of keratin fibres, in particular human hair, or for the treatment of skin.

17. A cosmetic agent according to one of the preceding claims 11 to 13, **characterised in that** it concerns an agent for influencing body odour, in particular a deodorising agent.

18. A method for prolonging the scent perception of washing or cleaning agents, fabric softeners or cosmetics or of solid surfaces treated with them, **characterised in that** compounds according to one of the claims 1 to 3 or mixtures according to the claim 4 are incorporated into the washing or cleaning agents, fabric softeners or cosmetics.

19. The method according to claim 18, **characterised in that** the scent is released again by hydrolysis.

## Revendications

1. Composé de 1-aza-3,7-dioxabicyclo[3.3.0]octane répondant à la formule générale (I) 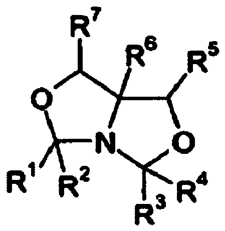 dans laquelle
R¹, R², R³, R⁴ représentent, indépendamment les uns des autres, des résidus qui procurent, dans un composé répondant à la formule générale R¹C(=O)-R² respectivement R³-C(=O)-R⁴, un aldéhyde odoriférant ou une cétone odoriférante, R¹ et R² respectivement R³ et R⁴ ne pouvant pas représenter simultanément un atome d'hydrogène ;
R⁶ représente un atome d'hydrogène, un groupe alkyle qui peut être substitué par deux groupes hydroxyle et/ou par un groupe amino et/ou dans lequel jusqu'à 8 groupes -CH₂- non voisins peuvent être remplacés par un atome d'oxygène,
R⁵, R⁷ représentent, indépendamment l'un de l'autre, un atome d'hydrogène ou un groupe alkyle en C₁-C₆ ;
ou mélanges de ces composés,
la cétone mentionnée étant choisie parmi le groupe constitué par la buccoxime ; l'isojasmone ; la méthyl β-naphtyl cétone ; le musc indanone ; le Tonalid/Musc plus ; l'α-damascone, la β-damascone, la δ-damascone, l'iso-damascone, la damascénone, la damarose, le méthyl-dihydrojasmonate, la menthone, la carvone, le camphre, la fenchone, l'α-ionone, la β-ionone, la dihydro-β-ionone, la pseudo γ-méthylionone, la fleuramone, la dihydrojasmone, la cis-jasmone, l'iso-E-Super, la méthyl-cédrényl-cétone ou la méthyl-cédrylone, l'acétophénone, la méthyl-acétophénone, la para-méthoxy-acétophénone, la méthyl-β-naphtyl-cétone, la benzyl-acétone, la benzophénone, la para hydroxyphényl butanone, la cétone de céleri ou la livescone, la 6-isopropyldécahydro-2-naphtone, la diméthyl-octénone, la freskomenthe, la 4-(1-éthoxyvinyl)-3,3,5,5,-tétraméthyl-cyclohexanone, la méthyl-hepténone, la 2-(2-(4-méthyl-3-cyclohexén-1-yl)propyl)cyclopentanone, la 1-(p-menthén-6(2)-yl)-1-propanone, la 4-(4-hydroxy-3-méthoxyphényl)-2-butanone, le 2-acétyl-3,3-diméthyl-norbornane, la 6,7-dihydro-1,1,2,3,3-pentaméthyl-4(5H)-indanone, le 4-damascol, le Dulcinyl ou le Cassion, la gelsone, l'hexalone, l'isocyclémone E, la méthyl-cyclocitrone, la méthyl-lavande-cétone, l'orivone, la para-tert-butylcyclohexanone, la verdone, la delphone, la muscone, la néobuténone, la plicatone, la veloutone, la 2,4,4,7-tétraméthyl-oct-6-én-3-one, le tétramérane, l'hédione et leurs mélanges ; et l'aldéhyde mentionné étant choisi parmi le mélonal, le triplal, le ligustral, l'adoxal ; l'anisaldéhyde ; le cymal ; l'éthylvanilline ; le florhydral ; l'hélional ; l'héliotropine ; l'hydroxycitronellal ; la koavone ; le laurinaldéhyde ; le lyral ; le méthyl-nonyl-acétaldéhyde ; le p,t-bucinal ; le phénylacétaldéhyde ; l'undécylènaldéhyde ; la vanilline ; le 2,6,10-triméthyl-9-undécénal, le 3-dodécén-1-al, l'α-n-amylcinnamaldéhyde, le 4-méthoxybenzaldéhyde, le benzaldéhyde, le 3-(4-tert-butylphényl)-propanal, le 2-méthyl-3-(para-méthoxyphénylpropanal), le 2-méthyl-4(2,6,6-triméthyl-2(1)-cyclohexén-1-yl)butanal, le 3-phényl-2-propénal, le cis-/trans-3,7-diméthyl-2,6-octadién-1-al, le 3,7-diméthyl-6-octén-1-al, le [(3,7-diméthyl-6-octényl)oxy]acétaldéhyde, le 4-isopropylbenzyaldéhyde, le 1,2,3,4,5,6,7,8-octahydro-8,8-diméthyl-2-naphtaldéhyde, le 2,4-diméthyl-3-cyclohexène-1-carboxyaldéhyde, le 2-méthyl-3-(isopropylphényl)propanal, le décylaldéhyde, le 2,6-diméthyl-5-hepténal ; le 4-(tricyclo[5.2.1.0(2,6)]-décylidène-8)-butanal ; l'octahydro-4,7-méthano-1H-indènecarboxaldéhyde ; le 3-éthoxy-4-hydroxybenzaldéhyde, le para-éthyl-α,α-diméthylhydrocinnamaldéhyde, l'α-méthyl-3,4-(méthylènedioxy)-hydrocinnamaldéhyde, le 3,4-méthylènedioxybenzaldéhyde, l'α-n-hexylcinnamaldéhyde, le m-cymène-7-carboxaldéhyde, l'α-méthylphénylacétaldéhyde, le 7-hydroxy-3,7-diméthyloctanal, l'undécénal, le 2,4,6-triméthyl-3-cyclohexène-1-carboxaldéhyde, le 4-(3)(4-méthyl-3-pentényl)-3-cyclohexène-carboxaldéhyde, le 1-dodécanal, le 2,4-diméthylcyclohexène-3-carboxaldéhyde, le 4-(4-hydroxy-4-méthylpentyl)-3-cylohexène-1-carboxaldéhyde, le 7-méthoxy-3,7-diméthyloctan-1-al, le 2-méthylundécanal, le 2-méthyldécanal, le 1-nonanal, le 1-octanal, le 2,6,10-triméthyl-5,9-undécadiénal, le 2-méthyl-3-(4-tert-butyl)propanal, le dihydrocinnamaldéhyde, le 1-méthyl-4-(4-méthyl-3-pentényl)-3-cyclohexène-1-carboxaldéhyde, le 5 ou 6 méthoxyhexahydro-4,7-méthanoindane-1- ou 2-carboxyaldéhyde ; le 3,7-diméthyloctan-1-al, le 1-undécanal, le 10-undécén-1-al, le 4-hydroxy-3-méthoxybenzaldéhyde, le 1-méthyl-3-(4-méthylpentyl)-3-cyclohexène-carboxyaldéhyde, le 7-hydroxy-3,7-diméthyloctanal ; le trans-4-décénal, le 2,6-nonadiénal, le para-tolylacétaldéhyde ; le 4-méthylphénylacétaldéhyde, le 2-méthyl-4-(2,6,6-triméthyl-1 cyclohexén-1-yl)-2-buténal, l'ortho-méthoxy-cinnamaldéhyde, le 3,5,6-triméthyl-3-cyclohexène-carboxaldéhyde ; le 3,7-diméthyl-2-méthylène-6-octénal, le phénoxyacétaldéhyde ; le 5,9-diméthyl-4,8-decadiénal, le pivoine-aldéhyde (6,10-diméthyl-3-oxa-5,9-undécadién-1-al), l'hexahydro-4,7-méthano-indane-1-carboxaldéhyde, le 2-méthyloctanal, l'α-méthyl-4-(1-méthyléthyl)benzène-acétaldéhyde, le 6,6-diméthyl-2-norpinène-2-propionaldéhyde, le para-méthyl phénoxy acétaldéhyde ; le 2-méthyl-3-phényl-2-propén-1-al, le 3,5,5-triméthylhexanal, l'hexahydro-8,8-diméthyl-2-naphtaldéhyde, le 3-propyl-bicyclo[2.2.1]-hept-5-ène-2-carbaldéhyde, le 9-décénal, le 3-méthyl-5-phényl-1-pentanal, le méthylnonylacétaldéhyde, le 1-p-menthène-q-carboxaldéhyde, le citral ou leurs mélanges, le lilial, le citral, le 1-décanal, le florhydral, le 2,4-diméthyl-3-cyclohexène-1-carboxaldéhyde.

2. Composé selon la revendication 1, **caractérisé en ce que**, au maximum dans un des éléments de structure -CR¹R² respectivement -CR³R⁴, sont présents des résidus R¹ et R² respectivement R³ et R⁴ qui procurent, dans un composé répondant à la formule générale R¹C(=O)-R² respectivement R³-C(=O)-R⁴, une cétone odoriférante.

3. Composé selon l'une quelconque des revendications 1 ou 2, **caractérisé en ce que** R², R⁴, R⁵, R⁶, R⁷ représentent un atome d'hydrogène et R¹ et R³ représentent respectivement un résidu d'hydrocarbure en C₄-C₂₄.

4. Mélange de composés répondant à la formule générale (I) selon l'une quelconque des revendications 1 à 3 et de composés répondant à la formule générale (II) 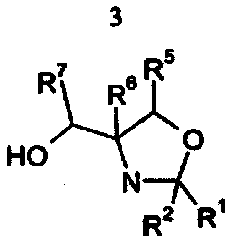 dans laquelle R¹, R², R⁵, R⁶, R⁷ ont les significations indiquées dans la formule générale (I).

5. Procédé pour la préparation de composés selon l'une quelconque des revendications 1 à 3 ou de mélanges selon la revendication 4 par mise en réaction de composés répondant à la formule générale (III) 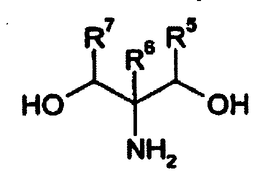 avec des composés répondant aux formules générales R¹C(=O)-R² et R³-C(=O)-R⁴, donnant lieu à une cyclisation.

6. Utilisation de composés selon l'une quelconque des revendications 1 à 3 ou de mélanges selon la revendication 4, à titre de précurseurs de parfums.

7. Utilisation selon la revendication 6, **caractérisée en ce que** les composés libèrent des aldéhydes odoriférants à titre de parfums.

8. Utilisation selon la revendication 6, **caractérisée en ce que** les composés libèrent des cétones odoriférantes à titre de parfums.

9. Utilisation selon l'une quelconque des revendications précédentes 6 à 8, **caractérisée en ce que** les composés sont mis en oeuvre conjointement avec d'autres parfums.

10. Utilisation selon l'une quelconque des revendications précédentes 6 à 9, dans des agents de lavage et de nettoyage, dans des agents adoucissants et dans des agents cosmétiques.

11. Agent de lavage ou de nettoyage, agent adoucissant ou agent cosmétique, contenant des composés selon l'une quelconque des revendications 1 à 3 ou des mélanges selon la revendication 4.

12. Agent de lavage ou de nettoyage, agent adoucissant ou agent cosmétique selon la revendication 11, **caractérisé en ce que** l'agent contient un composé de 1-aza-3,7-dioxabicyclo[3.3.0]octane ou des mélanges de ce dernier dans des quantités inférieures à 5 % en poids, de préférence inférieures à 2 % en poids, en particulier inférieures à 1 % en poids, chaque fois rapportés à la quantité totale des agents.

13. Agent de lavage ou de nettoyage, agent adoucissant ou agent cosmétique selon l'une quelconque des revendications 11 ou 12, **caractérisé en ce qu'**il s'agit de formulations solides, liquides ou sous forme de gels.

14. Agent de lavage ou de nettoyage selon la revendication 13, **caractérisé en ce que**, dans le cas de formulations solides, il s'agit de forme de poudres, de produits de granulation, de comprimés ou de pastilles, et dans le cas de formulations liquides, il s'agit de solutions, d'émulsions ou de dispersions.

15. Agent de lavage ou de nettoyage selon l'une quelconque des revendications 11 à 14, **caractérisé en ce qu'**il s'agit de détergents choisi parmi le groupe des détergents liquides ou sous forme de gels pour des surfaces dures, en particulier de ce qu'il est convenu d'appeler des détergents tous usages, des détergents pour le verre, des détergents pour les sols ou les salles de bains, ainsi que des formes de réalisation spéciales de détergents de ce type dont font partie des formes acides ou alcalines de détergents tous usages, ainsi que des détergents pour le verre possédant ce que l'on appelle un effet antipluie.

16. Agent cosmétique selon l'une quelconque des revendications 11 à 13, **caractérisé en ce qu'**il s'agit d'une préparation aqueuse qui contient des substances tensioactives et qui est appropriée en particulier pour le traitement de fibres kératiniques, en particulier des cheveux humains, ou bien pour le traitement de la peau.

17. Agent cosmétique selon l'une quelconque des revendications 11 à 13, **caractérisée en ce qu'**il s'agit d'un agent destiné à influencer l'odeur corporelle, en particulier d'un désodorisant.

18. Procédé pour prolonger la sensation odoriférante des agents de lavage ou de nettoyage, des agents adoucissants ou des agents cosmétiques ou bien des surfaces solides traitées avec les agents que l'on vient de citer, **caractérisé en ce qu'**on incorpore aux agents de lavage ou de nettoyage, aux agents adoucissants ou aux agents cosmétiques, des composés selon l'une quelconque des revendications 1 à 3 ou des mélanges selon la revendication 4.

19. Procédé selon la revendication 18, dans lequel le parfum est libéré par la suite par hydrolyse.
